# EUROPEAN PATENT APPLICATION

(11) **EP 4 063 350 A1**
(43) Date of publication of application: **28.09.2022**
(21) Application number: 20890768.3
(22) Date of filing: 20.11.2020
(51) Int. Cl.: C07D 209/52, C07D 209/02, C07D 209/00, C07D 221/04, C07D 491/048, C07D 401/04, A61K 31/403, A61K 31/407, A61K 31/438, A61P 5/26

(54) **BICYCLIC COMPOUND USED AS SELECTIVE ANDROGEN RECEPTOR MODULATOR**

(30) Priority: 20.11.2019 CN 201911143016; 30.07.2020 CN 202010750140
(71) Applicant: Medshine Discovery Inc., Nanjing, Jiangsu 210032 (CN)
(72) Inventor: SHEN, Chunli, Shanghai 200131 (CN); SUN, Guanglong, Shanghai 200131 (CN); WU, Chengde, Shanghai 200131 (CN); CHEN, Shuhui, Shanghai 200131 (CN)
(74) Representative: Dehns
(86) International application number: PCT/CN2020/130309
(87) International publication number: WO 2021/098809

(57) **Abstract**

Disclosed are a bicyclic compound used as a non-steroidal selective androgen receptor modulator and the use thereof in the preparation of a drug for treating related diseases which are mediated by an androgen receptor. Specifically, the present invention discloses a compound as shown in formula (I) or a pharmaceutically acceptable salt thereof.

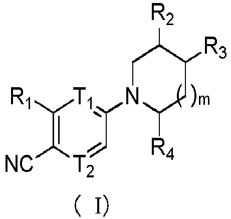

## Description

**The present application claims the right of the following priorities for:**
CN201911143016.7; application date: November 20, 2019;
CN202010750140.6; application date: July 30, 2020.

### TECHNICAL FIELD

The present disclosure relates to a class of bicyclic compounds used as non-steroidal-selective androgen receptor modulators, and use thereof in the manufacture of a medicament for the treatment of related diseases mediated by androgen receptor. The present disclosure specifically relates to a compound represented by formula (I) or a pharmaceutically acceptable salt thereof.

### BACKGROUND

Androgen receptor (AR), also known as NR3C4, belongs to the steroid receptor in the nuclear receptor superfamily; by combining with androgens, the androgen receptor can stimulate protein synthesis and metabolism, strengthen muscles and bones, and maintain hormone balance in the body, and the androgen receptor is an intermediary substance for androgen to play an important physiological role. With the increase of age, the production of androgen in the human body decreases progressively, and age-related diseases occur, such as muscle atrophy, cachexia, osteoporosis, fracture, fatigue and weakness, hypogonadism and other muscle and bone diseases; although the corresponding androgen therapy can regulate the growth of muscle and bone by activating androgen receptor and alleviate a series of androgen deficiency, it is easy to cause adverse side effects, such as acne, masculinization, excessive facial and body hair, prostatic hyperplasia and hypertrophy, cardiovascular related diseases, etc. Non-steroidal-selective androgen receptor modulators (SARMs), as partial agonists of androgen receptors, can selectively stimulate the anabolic pathways of androgen receptors in muscle and bone, increase the number and thickness of muscle fibers, enhance bone density and bone strength, and accelerate fracture recovery, thus effectively treating a variety of age-related diseases such as muscle atrophy and fractures, and avoiding serious side effects with a high therapeutic index. VK5211 (WO2009082437), a non-steroidal-selective androgen receptor modulator developed by Viking Therapeutics, is in clinical phase II.

In view of the important role of selective androgen receptor modulators, it is particularly important to develop selective androgen receptor modulators suitable for use as therapeutic drugs.

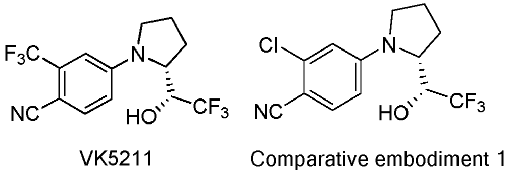

### CONTENT OF THE PRESENT INVENTION

The present disclosure provides a compound represented by formula (I) or a pharmaceutically acceptable salt thereof, 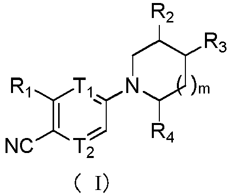 wherein,
T₁ is independently selected from N, CH and CR₅;
T₂ is independently selected from N, CH and CR₆;
R₁ is independently selected from H, F, Cl, Br, I, OH, NH₂, CN, C₁₋₃ alkyl and C₁₋₃ alkoxy, and the C₁₋₃ alkyl and C₁₋₃ alkoxy are optionally substituted by 1, 2 or 3 Rₐ;
R₂ and R₃ are each independently selected from F, Cl, Br, I, OH and NH₂;
alternatively, R₂ and R₃ combining with the atoms to which they are attached form C₃₋₅ cycloalkyl and tetrahydrofuranyl, and the C₃₋₅ cycloalkyl and tetrahydrofuranyl are optionally substituted by 1, 2 or 3 R_{b};
m is 0, 1 or 2;
R₄ is independently selected from F, Cl, Br, I, OH, C₁₋₆ alkyl and C₁₋₆ alkoxy, and the C₁₋₆ alkyl and C₁₋₆ alkoxy are optionally substituted by 1, 2 or 3 R_{c};
R₅ is independently selected from F, Cl, Br, I, CN, C₁₋₃ alkyl and C₁₋₃ alkoxy, and the C₁₋₃ alkyl and C₁₋₃ alkoxy are optionally substituted by 1, 2 or 3 R_{d};
R₆ is independently selected from F, Cl, Br, I, OH, NH₂ and CN;
Rₐ, R_{b} and R_{d} are each independently selected from F, Cl, Br, I and OH;
R_{c} is independently selected from F, Cl, Br, I, OH, C₁₋₃ alkyl and C₁₋₃ alkoxy, and the C₁₋₃ alkyl and C₁₋₃ alkoxy are optionally substituted by 1, 2 or 3 R;
R is independently selected from F, Cl, Br and I.

In some embodiments of the present disclosure, the R₁ is independently selected from H, F, Cl, Br, I, OH, NH₂, CN, CH₃, CH₂CH₃, C(CH₃)₂ and OCH₃, and the CH₃, CH₂CH₃, C(CH₃)₂ and OCH₃ are optionally substituted by 1, 2 or 3 Rₐ, and other variables are as defined in the present disclosure.

In some embodiments of the present disclosure, the R₁ is independently selected from H, F, Cl, Br, I, OH, NH₂, CN, CH₃, CH₂F, CHF₂, CF₃, CH₂CH₃, C(CH₃)₂, OCH₃ and OCHF₂, and other variables are as defined in the present disclosure.

In some embodiments of the present disclosure, the R₂ and R₃ combining with the atoms to which they are attached form cyclopropyl, cyclobutyl, cyclopentyl and tetrahydrofuranyl, and the cyclopropyl, cyclobutyl, cyclopentyl and tetrahydrofuranyl are optionally substituted by 1, 2 or 3 R_{b}, and other variables are as defined in the present disclosure.

In some embodiments of the present disclosure, the R₂ and R₃ combining with the atoms to which they are attached form 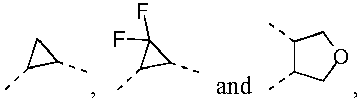 and other variables are as defined in the present disclosure.

In some embodiments of the present disclosure, the R_{c} is independently selected from F, Cl, Br, I, OH, CH₃, CH₂F, CHF₂, CF₃, CH₂CH₃, C(CH₃)₂ and OCH₃, and other variables are as defined in the present disclosure.

In some embodiments of the present disclosure, the R₄ is independently selected from F, Cl, Br, I, OH, C₁₋₃ alkyl and C₁₋₃ alkoxy, and the C₁₋₃ alkyl and C₁₋₃ alkoxy are optionally substituted by 1, 2 or 3 R_{c}, and other variables are as defined in the present disclosure.

In some embodiments of the present disclosure, the R₄ is independently selected from F, Cl, Br, I, OH, CH₃, CH₂CH₃ and OCH₃, and the CH₃, CH₂CH₃ and OCH₃ are optionally substituted by 1, 2 or 3 R_{c}, and other variables are as defined in the present disclosure.

In some embodiments of the present disclosure, the R₄ is independently selected from F, Cl, Br, I, OH, CH₃, CF₃, CH₂CH₃, OCH₃ and 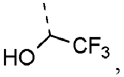 and other variables are as defined in the present disclosure.

In some embodiments of the present disclosure, the R₄ is independently selected from 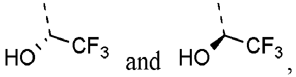 and other variables are as defined in the present disclosure.

In some embodiments of the present disclosure, the R₅ is independently selected from F, Cl, Br, I, CN, CH₃ and OCH₃, and other variables are as defined in the present disclosure.

In some embodiments of the present disclosure, the R₅ is independently selected from F, Cl, Br, I, CN, CH₃ and OCH₃, and other variables are as defined in the present disclosure.

In some embodiments of the present disclosure, the structural moiety 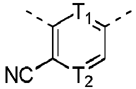 is selected from 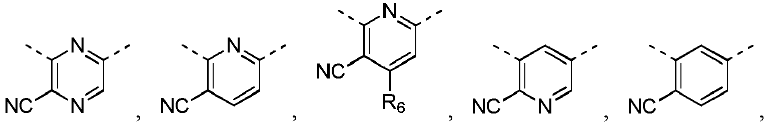 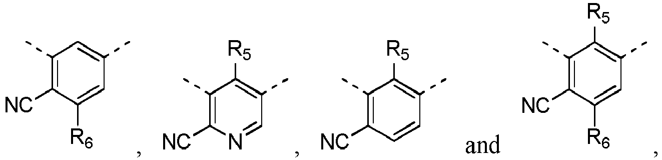 and other variables are as defined in the present disclosure.

In some embodiments of the present disclosure, the structural moiety 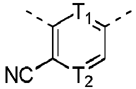 is selected from 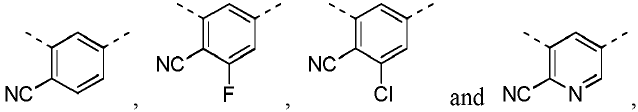 and the other variables are as defined in the present disclosure.

There are still some embodiments of the present disclosure which are obtained by any combination of the above variables.

In some embodiments of the present disclosure, the compound or the pharmaceutically acceptable salt thereof is selected from: 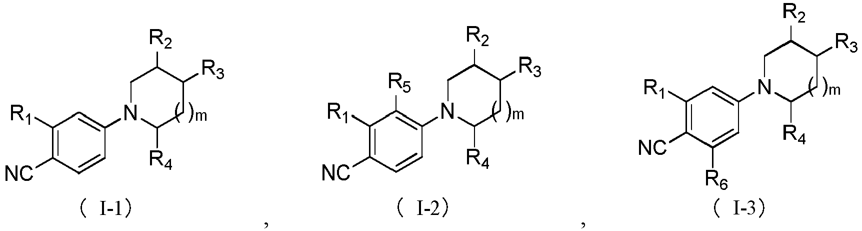 and 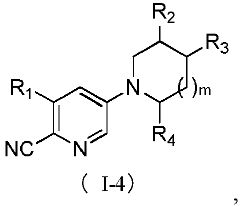 wherein,
R₁, R₂, R₃, R₄, R₅, R₆ and m are as defined in the present disclosure.

In some embodiments of the present disclosure, the compound or the pharmaceutically acceptable salt thereof is selected from: 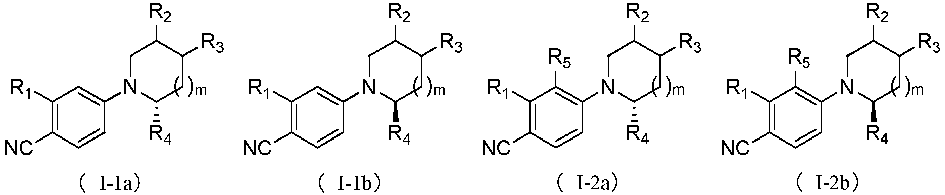 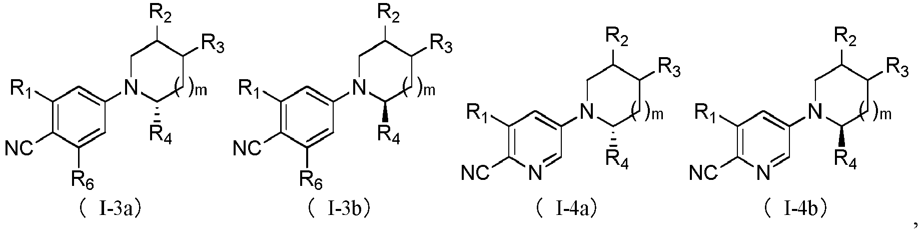 wherein,
R₁, R₂, R₃, R₄, R₅, R₆ and m are as defined in the present disclosure.

The present disclosure also provides a compound represented by the following formula or a pharmaceutically acceptable salt thereof,

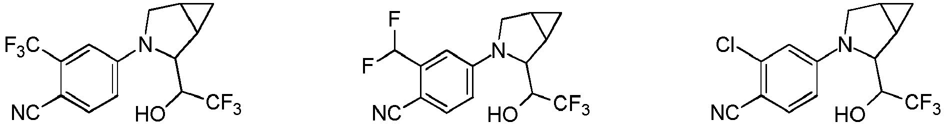

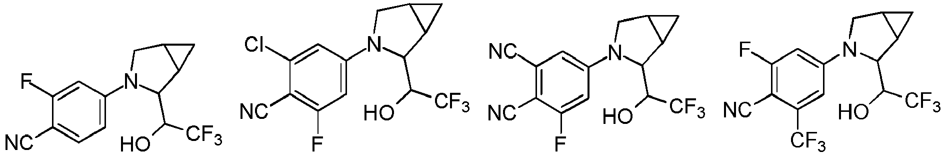

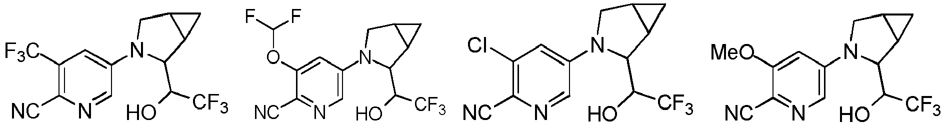

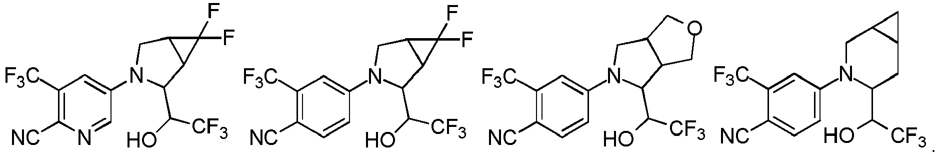

In some embodiments of the present disclosure, the compound or the pharmaceutically acceptable salt thereof is selected from:

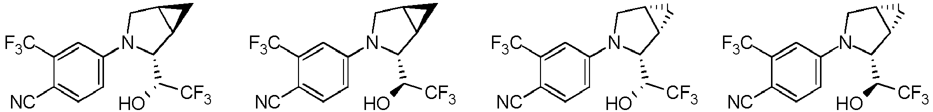

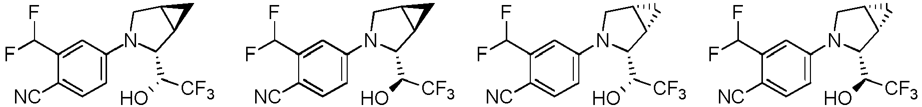

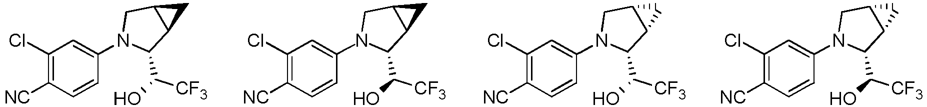

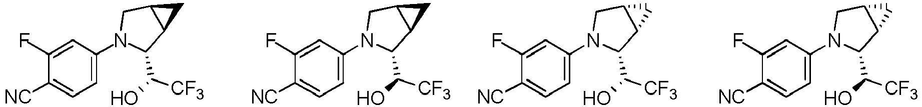

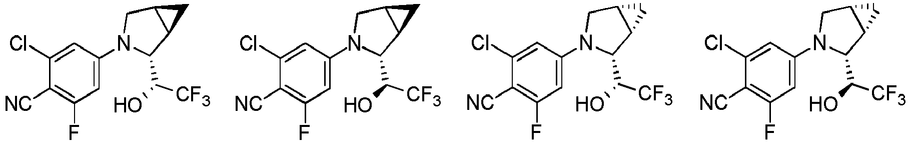

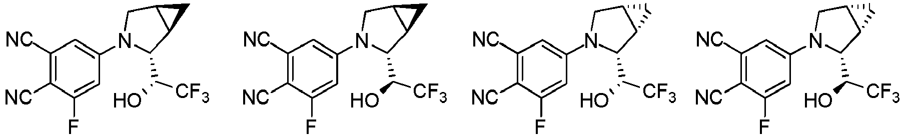

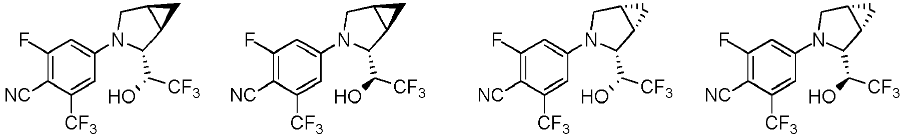

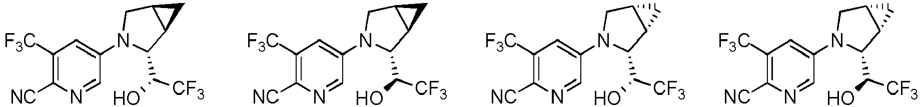

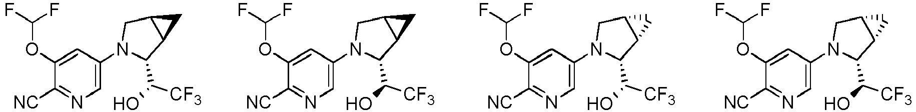

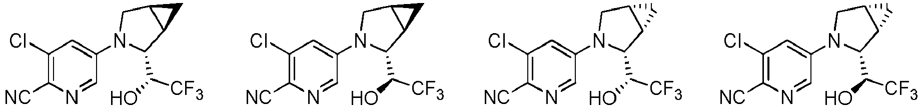

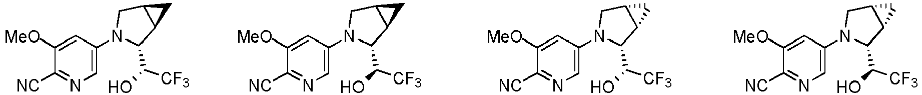

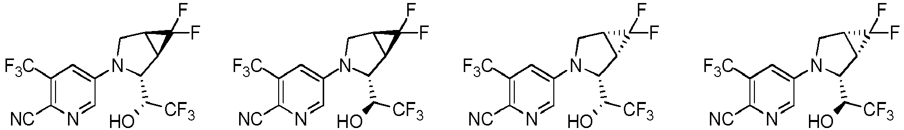

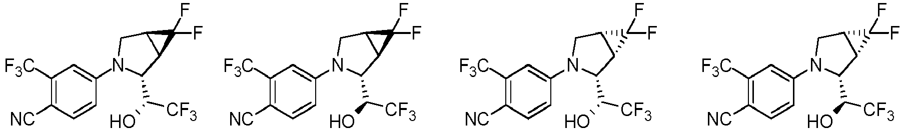

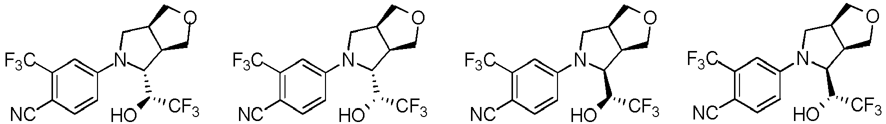

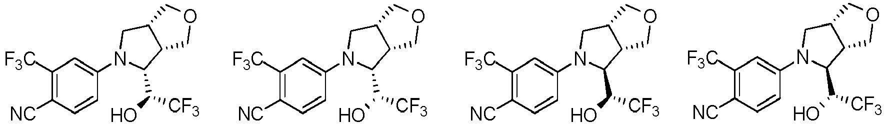

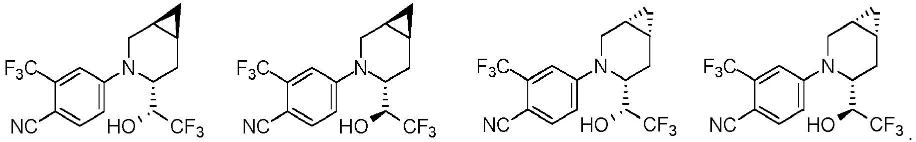

In some embodiments of the present disclosure, use of the compound or the pharmaceutically acceptable salt thereof in the manufacture of a medicament for the treatment of related diseases mediated by androgen receptor.

In some embodiments of the present disclosure, the above use is characterized in that, the medicament is a non-steroidal-selective androgen receptor modulator medicament.

In some embodiments of the present disclosure, the above use is characterized in that, the medicament is a medicament for various senile diseases such as muscle atrophy, fracture, osteoporosis and the like.

### Technical effect

The compounds of the present disclosure exhibit significant selective androgen receptor modulating activity. The compounds of the present disclosure have long oral half-life, a certain oral exposure and oral bioavailability, good oral PK properties, and exhibit significant body weight and muscle weight gain effects on complete female animal models with less side effects on gonadal organs. The compounds of the present disclosure have low risk of drug combination.

### Definition and description

Unless otherwise stated, the following terms and phrases used herein have the following meanings. A specific term or phrase should not be considered indefinite or unclear in the absence of a particular definition, but should be understood according to the common meaning. When a trade name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the neutral form of the compound with a sufficient amount of a base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by contacting the neutral form of the compound with a sufficient amount of an acid in a pure solution or a suitable inert solvent. Examples of the pharmaceutically acceptable acid addition salts include salts derived from inorganic acids, such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to either base or acid addition salts.

The pharmaceutically acceptable salt of the present disclosure can be prepared from the parent compound that contains an acidic or basic group by conventional chemical methods. Generally, such salt can be prepared by reacting the free acid or base form of the compound with a stoichiometric amount of an appropriate base or acid in water or an organic solvent or a mixture thereof.

Unless otherwise specified, the term "isomer" is intended to include geometric isomers, *cis-trans* isomers, stereoisomers, enantiomers, optical isomers, diastereoisomers, and tautomers.

The compounds of the present disclosure may exist in specific geometric or stereoisomeric forms. The present disclosure contemplates all such compounds, including *cis* and *trans* isomers, (-)-and (+)-enantiomers, (*R*)-and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" result form the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 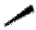 ) and a wedged dashed bond ( 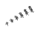 ), and the relative configuration of a stereogenic center is represented by a straight solid bond ( 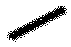 ) and a straight dashed bond ( 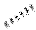 ), a wave line ( 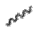 ) is used to represent a wedged solid bond ( 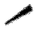 ) or a wedged dashed bond ( 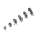 ), or the wave line ( 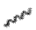 ) is used to represent a straight solid bond ( 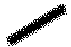 ) or a straight dashed bond ( 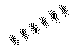 ).

Unless otherwise specified, the terms "enriched in one isomer", "enriched in isomers", "enriched in one enantiomer" or "enriched in enantiomers" refer to the content of one of the isomers or enantiomers is less than 100 %, and the content of the isomer or enantiomer is greater than or equal to 60 %, or greater than or equal to 70 %, or greater than or equal to 80 %, or greater than or equal to 90 %, or greater than or equal to 95 %, or greater than or equal to 96 %, or greater than or equal to 97 %, or greater than or equal to 98 %, or greater than or equal to 99 %, or greater than or equal to 99.5 %, or greater than or equal to 99.6 %, or greater than or equal to 99.7 %, or greater than or equal to 99.8 %, or greater than or equal to 99.9 %.

Unless otherwise specified, the term "isomer excess" or "enantiomeric excess" refers to the difference between the relative percentages of two isomers or two enantiomers. For example, if the content of one isomer or enantiomer is 90 %, and the content of the other isomer or enantiomer is 10 %, the isomer or enantiomer excess (ee value) is 80 %.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is firmer than that of ordinary hydrogen and carbon, compared with non-deuterated drugs, deuterated drugs have the advantages of reduced toxic and side effects, increased drug stability, enhanced efficacy, prolonged biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond.

When the number of a substituent is 0, it means that the substituent does not exist, for example, -A-(R)o means that the structure is actually -A.

When a substituent is vacant, it means that the substituent does not exist, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such a substituent can be bonded to any atom on the ring, for example, a structural moiety 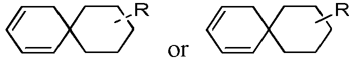 indicates that its substituent R can be substituted at any position on cyclohexyl or cyclohexene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 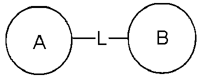 is -M-W-, then -M-W- can link ring A and ring B to form 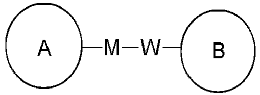 in the direction same as left-to-right reading order, and form 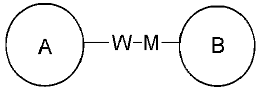 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of chemical bond linking thereto so as to meet the corresponding valence. The chemical bond between the site and other groups can be represented by a straight solid bond 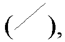 a straight dashed bond 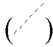 or a wavy line 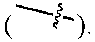 For example, the straight solid bond in -OCH₃ means that it is linked to other groups through the oxygen atom in the group; the straight dashed bonds in 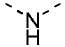 means that it is linked to other groups through the two ends of nitrogen atom in the group; the wave lines in 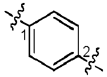 means that the phenyl group is linked to other groups through carbon atoms at position 1 and position 2; 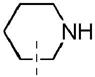 means that it can be linked to other groups through any linkable sites on the piperidinyl by one chemical bond, including at least four types of linkage, including 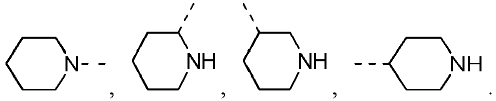 Even though the H atom is drawn on the -N-, 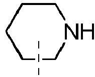 still includes the linkage of 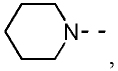 merely when one chemical bond was connected, the H of this site will be reduced by one to the corresponding monovalent piperidinyl.

Unless otherwise specified, the number of atoms in a ring is generally defined as the number of ring members, e.g., "5- to 7-membered ring" refers to a "ring" of 5-7 atoms arranged around it.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆ and C₅ alkyl, etc. It can be monovalent (such as methyl), divalent (such as methylene) or multivalent (such as methine). Examples of C₁₋₆ alkyl include, but are not limited to methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl and neopentyl), hexyl, etc.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene) or multivalent (such as methine). Examples of C₁₋₃ alkyl include, but are not limited to methyl (Me), ethyl (Et), propyl (including *n*-propyl and isopropyl), etc.

Unless otherwise specified, the term "C₁₋₆ alkoxy" refers to an alkyl group containing 1 to 6 carbon atoms that are connected to the rest of the molecule through an oxygen atom. The C₁₋₆ alkoxy includes C₁₋₄, C₁₋₃, C₁₋₂, C₂₋₆, C₂₋₄, C₆, C₅, C₄ and C₃ alkoxy, etc. Examples of C₁₋₆ alkoxy include, but are not limited to, methoxy, ethoxy, propoxy (including *n*-propoxy and isopropoxy), butoxy (including *n*-butoxy, isobutoxy, s-butoxy, and *t*-butoxy), pentyloxy (including *n*-pentyloxy, isopentyloxy, and neopentyloxy), and hexyloxy, etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃ and C₂ alkoxy, etc. Examples of C₁₋₃ alkoxy include, but are not limited to, methoxy, ethoxy, propoxy (including *n*-propoxy and isopropoxy), etc.

Unless otherwise specified, "C₃₋₅ cycloalkyl" refers to a saturated cyclic hydrocarbon group composed of 3 to 5 carbon atoms, which is a monocyclic system, and the C₃₋₅ cycloalkyl includes C₃₋₄ and C₄₋₅ cycloalkyl; it can be monovalent, divalent or polyvalent. Examples of C₃₋₅ cycloalkyl include, but are not limited to, cyclopropyl, cyclobutyl, cyclopentyl, etc.

Unless otherwise specified, the term "3- to 6-membered heterocycloalkyl" by itself or in combination with other terms refers to a saturated cyclic group consisting of 3 to 6 ring atoms, wherein 1, 2, 3 or 4 ring atoms are heteroatoms independently selected from O, S and N, and the rest are carbon atoms, wherein nitrogen atoms are optionally quaternized, and nitrogen and sulfur heteroatoms can be optionally oxidized (i.e., NO and S(O)ₚ, p is 1 or 2). The 3- to 6-membered heterocycloalkyl includes monocyclic and bicyclic systems, wherein the bicyclic systems include spiro ring, fused ring and bridged ring. In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, and 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl or homopiperidinyl, etc.

The term "protecting group" includes, but is not limited to "amino protecting group", "hydroxyl protecting group" or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl or trifluoroacetyl); alkoxycarbonyl, such as *tert*-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and *tert-*butyldimethylsilyl (TBS), etc. The term "hydroxyl protecting group" refers to a protecting group suitable for preventing the side reactions of hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), *p*-methoxybenzyl (PMB), 9-fluorenylmethyl (Fm), and diphenylmethyl (benzhydryl, DPM); silyl, such as trimethylsilyl (TMS) and *tert*-butyl dimethyl silyl (TBS), etc.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The solvents used in the present disclosure are commercially available.

The present disclosure adopts the following abbreviation: aq represents water; eq represents equivalent; DCM represents dichloromethane; PE represents petroleum ether; DMSO represents dimethyl sulfoxide; EtOAc represents ethyl acetate; EtOH represents ethanol; MeOH represents methanol; Cbz represents benzyloxycarbonyl and is an amine protecting group; BOC represents *tert*-butoxycarbonyl and is an amine protecting group; r.t. represents room temperature; O/N represents overnight; THF represents tetrahydrofuran; Boc₂O represents di*tert*-butyldicarbonate; TFA represents trifluoroacetic acid; DIPEA represents diisopropylethylamine; iPrOH represents 2-propanol; mp represents melting point.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

### Embodiment 1

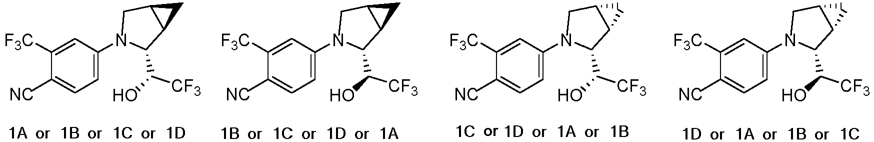

### Synthetic route:

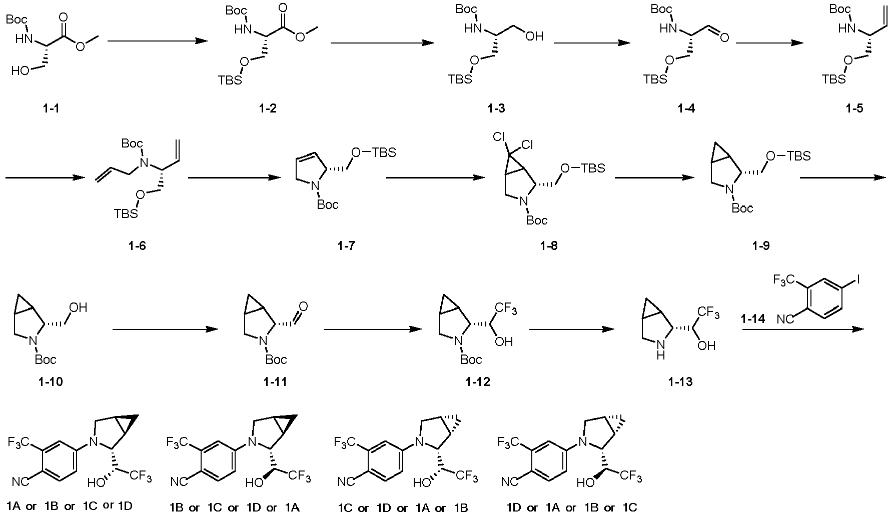

### 1) Synthesis of compound 1-2

Compound 1-1 (100 g, 456.13 mmol, 92.59 mL), dichloromethane (1000 mL) were added to a three-necked flask, and then *tert-*butyldimethylsilyl chloride (82.50 g, 547.36 mmol, 67.07 mL), imidazole (62.10 g, 912.26 mmol) were added thereto; the reaction system was replaced with nitrogen for three times, and the reaction was carried out at 25 °C for 16 hours. 1 M hydrochloric acid aqueous solution (1000 mL) was added to the system, and the organic phase was washed with saturated brine (1000 mL^{∗}2), dried over anhydrous sodium sulfate, filtered, and the filtrate was concentrated under reduced pressure to obtain crude compound 1-2. ¹HNMR (400 MHz, CDCl₃) δ ppm 5.34 (br d, *J*=9.41 Hz, 1H), 4.36 (br d, *J*=8.41 Hz, 1H), 4.05 (dd, *J*=10.10, 2.70 Hz, 1H), 3.83 (dd, *J*=10.04, 3.01 Hz, 1H), 3.75 (s, 3H) 1.47 (s, 9H), 0.87 (s, 9H), 0.01-0.06 (m, 6H).

### 2) Synthesis of compound 1-3

Compound 1-2 (172 g, 515.75 mmol), tetrahydrofuran (1.6 L) were added to a three-necked flask, and then lithium borohydride (16.85 g, 773.62 mmol) was added thereto, and the reaction was carried out at 25 °C for 7 hours. Saturated ammonium chloride solution (1500 mL) was added to the system and ethyl acetate (500 mL) was added for extraction, and the organic phase was collected, washed with saturated brine (500 mL^{∗}3), dried over anhydrous sodium sulfate, filtered, and the filtrate was concentrated under reduced pressure to obtain crude compound 1-3.

### 3) Synthesis of compound 1-4

Compound 1-3 (67.5 g, 220.96 mmol), *N*,*N*-diisopropylethylamine (85.67 g, 662.88 mmol, 115.46 mL), ethyl acetate (670 mL) were added to a three-necked flask, and the mixture was cooled to 0 °C, and a solution of pyridine sulfur trioxide complex (70.34 g, 441.92 mmol) in dimethyl sulfoxide (670 mL) was added thereto, and then the reaction was carried out at 0 °C for 2 hours. Ethyl acetate (1 L) was added to the reaction solution, and the mixture was washed with saturated brine solution (500 mL^{∗}3); the organic phase was collected, washed with 1 M hydrochloric acid aqueous solution (500 mL^{∗}3), and the organic phase was collected, dried over anhydrous sodium sulfate, filtered, and the filtrate was concentrated under reduced pressure to obtain crude compound 1-4.

### 4) Synthesis of compound 1-5

Compound methyltriphenylphosphonium bromide (215.27 g, 602.63 mmol), tetrahydrofuran (1000 mL) were added to a three-necked flask, and the mixture was cooled to 0 °C, then sodium bis(trimethylsilyl)amide (1 M, 920.69 mL) was added thereto; the system was stirred at 0 °C for 0.5 hours, then a solution of compound 1-4 (127 g, 418.49 mmol) in tetrahydrofuran (300 mL) was added thereto, and the system was naturally warmed to 15 °C and stirred for 15 hours. The system was quenched by adding saturated ammonium chloride (1000 mL) and extracted with ethyl acetate (1000 mL), and the organic phase was collected, washed with saturated brine (500 mL^{∗}3), dried over anhydrous sodium sulfate, filtered, and the filtrate was concentrated under reduced pressure to obtain a crude product, and the crude product was purified by column chromatography to obtain crude compound 1-5. LCMS (ESI) m/z: 246 [M-55]⁺.

### 5) Synthesis of compound 1-6

Compound 1-5 (110 g, 364.85 mmol), allyl bromide (88.28 g, 729.69 mmol), *N,N-*dimethylformamide (1L) were added to a pre-dried reaction flask, and the mixture was cooled to 0 °C, then a solution of potassium *tert*-butoxide (81.88 g, 729.69 mmol) in *N,N-*dimethylformamide (0.5 L) was added thereto, and the reaction was carried out at 0 °C for 2 hours. Water (700 mL) was added to the reaction system, and the mixture was extracted with ethyl acetate (700 mL), and the organic phase was collected, washed with saturated brine (350 mL^{∗}3), dried over anhydrous sodium sulfate, filtered, and the filtrate was concentrated under reduced pressure to obtain crude compound 1-6. LCMS (ESI) m/z: 242 [M-100+1]⁺.

### 6) Synthesis of compound 1-7

Compound 1-6 (27.5 g, 80.51 mmol), dichloromethane (300 mL), Grubb catalyst I (6.63 g, 8.05 mmol) were added to a round bottom flask, and the reaction system was replaced with nitrogen for three times, then the reaction was carried out at 25 °C for 48 hours. The system was concentrated to obtain a crude product, and the crude product was purified by column chromatography to obtain compound 1-7. ¹HNMR (400 MHz, CDCl₃) δ ppm 5.75-5.90 (m, 2H), 4.39-4.61 (m, 1H), 4.07-4.30 (m, 1H), 3.93-4.07 (m, 1H), 3.82-3.93 (m, 1H), 3.50-3.81 (m, 1H), 1.49 (d, *J*=6.02 Hz, 9H), 0.88 (br d, *J*=5.77 Hz, 9H), 0.01-0.07 (m, 6H).

### 7) Synthesis of compound 1-8

Compound 1-7 (42.5 g, 135.56 mmol), chloroform (800 mL), benzyltriethylammonium chloride (6.18 g, 27.11 mmol) were added to a pre-dried reaction flask, then 50 % sodium hydroxide aqueous solution (800 mL) was added dropwise thereto, and the system was stirred at 25 °C for 3 hours. Water (1000 mL) and dichloromethane (1000 mL) were added to the reaction solution, and the reaction solution was emulsified; after 1 M hydrochloric acid aqueous solution (400 mL) was added thereto, the reaction solution was passed through a funnel covered with diatomite, filtered under reduced pressure, and the phases of the filtrate were separated; the organic phase was collected, washed with saturated brine (100 mL^{∗}3), dried over anhydrous sodium sulfate, filtered, and the filtrate was concentrated under reduced pressure to obtain a crude product, then the crude product was purified by column chromatography to obtain compound 1-8. ¹HNMR (400 MHz, CDCl₃) δ ppm 3.92-4.09 (m, 1H), 3.81-3.88 (m, 1H), 3.74-3.80 (m, 1H), 3.54-3.73 (m, 2H), 2.19-2.39 (m, 2H), 1.44 (d, *J*=2.26 Hz, 9H), 0.90 (d, *J*=4.77 Hz, 9H), 0.06 (dd, *J*=7.65, 6.27 Hz, 6H).

### 8) Synthesis of compound 1-9

Sodium (23.20 g, 1.01 mol, 23.91 mL), tetrahydrofuran (400 mL) were added to a three-necked flask, then a solution of compound 1-8 (40 g, 100.90 mmol) in tetrahydrofuran (400 mL) and methanol (400 mL) was added dropwise thereto, and the system was stirred at 25 °C for 3 hours. Water (800 mL) was added to the reaction solution, and the mixture was extracted with ethyl acetate (800 mL^{∗}3); the organic phases were combined, washed with saturated brine (100 mL^{∗}3), dried over anhydrous sodium sulfate, filtered, and the filtrate was concentrated under reduced pressure to obtain a crude product, and the crude product was purified by column chromatography to obtain compound 1-9.

### 9) Synthesis of compound 1-10

Compound 1-9 (15 g, 45.80 mmol), tetrahydrofuran (150 mL) were added to a three-necked flask, and tetrabutylammonium fluoride (1 M, 68.70 mL) was added dropwise thereto, and the reaction was carried out at 25 °C for 2 hours. 1 M hydrochloric acid aqueous solution (300 mL) was added to the system, and the phases were separated, then the organic phase was washed with saturated brine (200 mL^{∗}3), dried over anhydrous sodium sulfate, filtered, and the filtrate was concentrated under reduced pressure to obtain a crude product, then the crude product was purified by column chromatography to obtain compound 1-10.

### 10) Synthesis of compound 1-11

Compound 1-10 (3.00 g, 14.07 mmol), ethyl acetate (30 mL), *N,N-*diisopropylethylamine (10.91 g, 84.40 mmol, 14.70 mL) were added to a three-necked flask, and the reaction system was replaced with nitrogen for three times, then the mixture was cooled to 0 °C; a solution of pyridine sulfur trioxide complex (6.72 g, 42.20 mmol) in dimethyl sulfoxide (30 mL) was added thereto, and the reaction was carried out at 0 °C for 2 hours. Ethyl acetate (50 mL) was added to the reaction solution, and the organic phase was washed with saturated brine solution (20 mL^{∗}3), and washed with 1 M hydrochloric acid aqueous solution (20 mL^{∗}3); the organic phase was dried over anhydrous sodium sulfate, filtered, and the filtrate was concentrated under reduced pressure to obtain crude compound 1-11. LCMS (ESI) m/z: 156 [M-55]⁺.

### 11) Synthesis of compound 1-12

Compound 1-11 (2.93 g, 13.87 mmol), (trifluoromethyl)trimethylsilane (2.96 g, 20.80 mmol), tetrahydrofuran (30 mL) were added to a three-necked flask, and the mixture was cooled to 0 °C; a solution of tetrabutylammonium fluoride in tetrahydrofuran (1 M, 27.74 mL) was added thereto, and the reaction was carried out at 0 °C for 2 hours. Ethyl acetate (30 mL) was added to the reaction solution, and the mixture was washed with 1 M hydrochloric acid aqueous solution (30 mL^{∗}3); the organic phase was dried over anhydrous sodium sulfate, filtered, and the filtrate was concentrated under reduced pressure to obtain crude compound 1-12. LCMS (ESI) m/z: 226 [M-55]⁺.

### 12) Synthesis of compound 1-13

Compound 1-12 (3.50 g, 12.44 mmol), hydrochloric acid/ethyl acetate (6M, 30 mL) were added to a round bottom flask, and the reaction system was replaced with nitrogen for three times, then the reaction was carried out at 25 °C for 1 hour. Water (10 mL) was added to the reaction solution, and ethyl acetate (20 mL) was added thereto, then the phases were separated, and the aqueous phase was collected. The pH of aqueous phase was adjusted to 11 with a saturated lithium hydroxide aqueous solution, then the mixture was extracted with ethyl acetate (50 mL^{∗}3); the organic phases were combined, washed with saturated brine (20 mL^{∗}2), dried over anhydrous sodium sulfate, filtered, and the filtrate was concentrated under reduced pressure to obtain crude compound 1-13.

### 13) Synthesis of compound 1A or 1B or 1C or 1D

Compound 1-13 (120 mg, 662.42 µmol), compound 1-14 (216.42 mg, 728.66 µmol), cesium carbonate (431 mg, 1.32 mmol), 2,2-bis(diphenylphosphino)-1,1-binaphthyl (41 mg, 66.24 µmol), tris(dibenzylideneacetone) dipalladium (60 mg, 66.24 µmol) were added to a pre-dried single-necked flask, then dioxane (3 mL) was added thereto, and the reaction system was evacuated and replaced with nitrogen for three times, then the mixture was stirred at 100 °C for 16 hours. After the reaction solution was concentrated under reduced pressure, ethyl acetate (20 mL) and saturated brine (10 mL) were added thereto, and the phases were separated; the organic phase was collected, dried over anhydrous sodium sulfate, filtered, and the filtrate was concentrated under reduced pressure to obtain a crude product, then the crude product was purified by preparative high performance liquid chromatography (alkaline system) and resolved by supercritical fluid chromatography (alkaline system) to obtain compound 1A. SFC detection (ee: 100 %), chromatographic column: Lµx Cellµlose-2 50 × 4.6 mm I.D., 3 µm; mobile phase: A: supercritical carbon dioxide, B: 0.05 % isopropanol solution of isopropylamine; gradient: from 5 % to 40 % in 1 minute, held 40 % for 1 min, returned to 5 % in 0.5 min, equilibrated at 5 % for 1.5 min; flow rate: 4 mL/min; column temperature: 35 °C; wavelength: 220 nm, retention time: 1.19 min. ¹HNMR (400 MHz, CDCl₃) δ ppm 7.58-7.49 (m, 1H), 6.81 (d, *J*=1.9 Hz, 1H), 6.63 (dd, *J*=2.2, 8.7 Hz, 1H), 4.39-4.08 (m, 2H), 3.69 (dd, *J*=4.0, 9.0 Hz, 1H), 3.51 (d, *J*=9.2 Hz, 1H), 2.73 (br s, 1H), 1.97-1.72 (m, 2H), 0.98-0.76 (m, 1H), 0.22 (q, *J*=4.2 Hz, 1H); LCMS (ESI) m/z: 351 [M+1]⁺.

### Embodiment 2

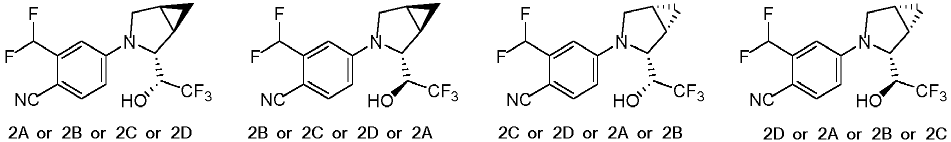

### Synthetic route:

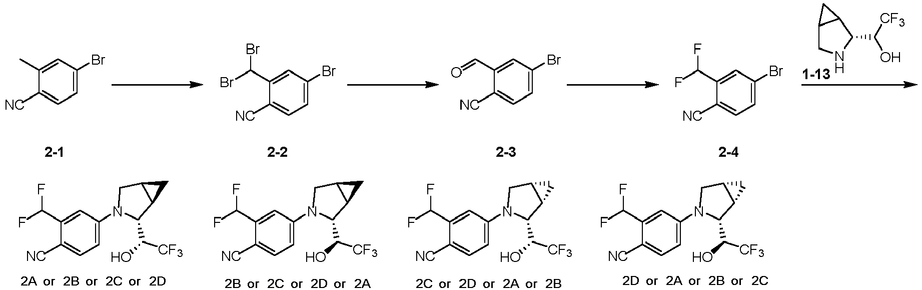

### 1) Synthesis of compound 2-2

Compound 2-1 (1 g, 5.10 mmol), *N*-bromosuccinimide (2 g, 11.22 mmol), benzoyl peroxide (185 mg, 765.13 µmol), carbon tetrachloride (10 mL) were added to a round bottom flask, and the reaction system was replaced with nitrogen for three times, then the reaction was carried out at 90 °C for 16 hours. The reaction solution was concentrated under reduced pressure to obtain a crude product, and the crude product was purified by column chromatography to obtain compound 2-2. ¹HNMR (400 MHz, CDCl₃) δ ppm 8.18 (d, *J*=1.88 Hz, 1H), 7.60 (dd, *J*=8.22, 1.82 Hz, 1H), 7.48 (d, *J*=8.28 Hz, 1H), 6.92 (s, 1H).

### 2) Synthesis of compound 2-3

Compound 2-2 (700 mg, 1.98 mmol), silver nitrate (1.68 g, 9.89 mmol), water (1 mL), and acetonitrile (8 mL) were added to a round bottom flask, and the reaction system was replaced with nitrogen for three times, then the reaction was carried out at 90 °C for 16 hours. The reaction solution was concentrated under reduced pressure to obtain a crude product, and the crude product was purified by column chromatography to obtain compound 2-3. ¹HNMR (400 MHz, CDCl₃) δ ppm 10.27 -10.36 (m, 1H), 8.19 (d, *J*=1.88 Hz, 1H), 7.90 (dd, *J*=8.16, 2.01 Hz, 1H), 7.71 (d, *J*=8.16 Hz, 1H).

### 3) Synthesis of compound 2-4

Compound 2-3 (400 mg, 1.90 mmol), ethanol (1.39 µL), dichloromethane (4 mL), and diethylaminosulfur trifluoride (614 mg, 3.81 mmol, 503.26 µL) were added to a thumb bottle, and the reaction system was replaced with nitrogen for three times, then the reaction was carried out at 25 °C for 1 hour. Saturated sodium bicarbonate solution (10 mL) was added to the reaction solution, then the mixture was extracted with dichloromethane (10 mL^{∗}3); the organic phases were collected, dried over anhydrous sodium sulfate, filtered, and the filtrate was concentrated under reduced pressure to obtain a crude product, and the crude product was purified by column chromatography to obtain compound 2-4. ¹HNMR (400 MHz, CDCl₃) δ ppm 7.93 (s, 1H), 7.78 (dd, *J*=8.28, 0.88 Hz, 1H), 7.63 (d, *J*=8.16 Hz, 1H), 6.74-7.06 (m, 1H).

### 4) Synthesis of compound 2A or 2B or 2C or 2D

Compound 1-13 (100 mg, 552.01 µmol), compound 2-4 (128 mg, 552.01 µmol), cesium carbonate (449 mg, 1.38 mmol), tris(dibenzylideneacetone)dipalladium (50 mg, 55.20 µmol), 2,2-bis(diphenylphosphino)-1,1-binaphthyl (34 mg, 55.20 µmol), and dioxane (2 mL) were added to a thumb bottle, and the reaction system was replaced with nitrogen for three times, then the reaction was carried out at 100 °C for 16 hours. The reaction solution was concentrated under reduced pressure to obtain a crude product, and the crude product was purified by column chromatography and preparative high performance liquid chromatography (alkaline system) to obtain a mixture of compound 2A and compound 2B. SFC detection (the ratio was 45: 55), chromatographic column: Chiralpak AS-3 150 × 4.6 mm I.D., 3 µm; mobile phase: A: supercritical carbon dioxide, B: 0.05 % ethanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: compound 2A (1.37 min) and compound 2B (1.71 min). ¹HNMR (400 MHz, CDCl₃) δ ppm 7.53 (d, *J*=8.77 Hz, 1H), 6.68-7.00 (m, 3H), 4.38 (d, *J*=5.70 Hz, 1H), 4.19 (br d, *J*=4.82 Hz, 1H), 3.59-3.68 (m, 1H), 3.51-3.57 (m, 1H), 2.38 (br s, 1H), 1.85 (br s, 1H), 1.70-1.79 (m, 1H), 0.82-0.91 (m, 1H), 0.10-0.17 (m, 1H); LCMS (ESI) m/z: 333 [M+1]⁺.

### Embodiment 3

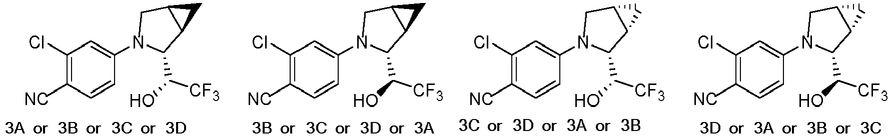

### Synthetic route:

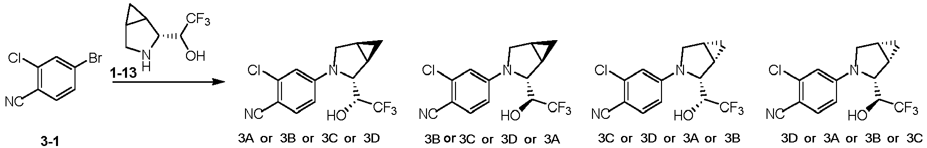

### 1) Synthesis of compound 3A or 3B or 3C or 3D

Compound 1-13 (200 mg, 1.10 mmol), compound 3-1 (238 mg, 1.10 mmol), cesium carbonate (719 mg, 2.21 mmol), 2,2-bis(diphenylphosphino)-1,1-binaphthyl (68 mg, 110.40 µmol), tris(dibenzylideneacetone) dipalladium (101 mg, 110.40 µmol) were added to a pre-dried single-necked flask, then 1,4-dioxane (4 mL) was added thereto, and the reaction system was evacuated and replaced with nitrogen for three times, then the mixture was stirred at 100 °C for 16 hours. The reaction solution was concentrated under reduced pressure to obtain a crude product, then the crude product was purified by preparative high performance liquid chromatography (alkaline system) and resolved by supercritical fluid chromatography (alkaline system) to obtain compound 3A. SFC detection (ee: 98.48 %), chromatographic column: Chiralpak AS-3 150 × 4.6 mm I.D., 3 µm; mobile phase: A: supercritical carbon dioxide, B: 0.05 % isopropanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 2.61 min. ¹HNMR (400 MHz, CDCl₃) δ ppm 7.40 (d, *J*=8.8 Hz, 1H), 6.56 (d, *J*=2.3 Hz, 1H), 6.42 (dd, *J*=2.4, 8.9 Hz, 1H), 4.31 (t, *J*=7.8 Hz, 1H), 4.23 (s, 1H), 3.64 (dd, *J*=4.0, 9.1 Hz, 1H), 3.44 (s, 1H), 2.53 (d, *J*=6.7 Hz, 1H), 1.93-1.74 (m, 2H), 0.84 (dt, *J*=5.5, 7.7 Hz, 1H), 0.26-0.13 (m, 1H); LCMS (ESI) m/z: 317 [M+1]⁺.

### Embodiment 4

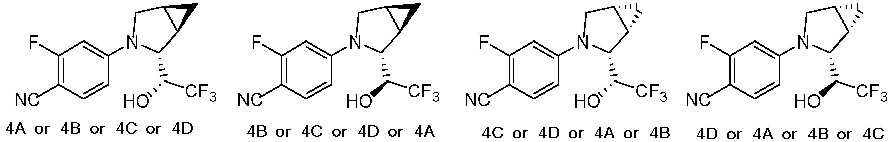

### Synthetic route:

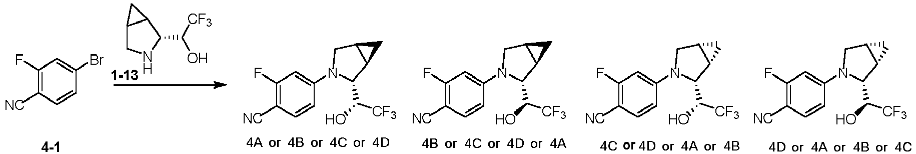

### 1) Synthesis of compound 4A or 4B or 4C or 4D

Compound 1-13 (30 mg, 165.60 µmol), compound 4-1 (33 mg, 165.60 µmol), potassium phosphate (105 mg, 496.81 µmol), tris(dibenzylideneacetone)dipalladium (15 mg, 16.56 µmol), 2,2-bis(diphenylphosphino)-1,1-binaphthyl (10 mg, 16.56 µmol), and dioxane (2 mL) were added to a thumb bottle, and the reaction system was replaced with nitrogen for three times, then the reaction was carried out at 100 °C for 16 hours. The reaction solution was concentrated under reduced pressure to obtain a crude product, then the crude product was purified by preparative high performance liquid chromatography (alkaline system) and resolved by supercritical fluid chromatography (alkaline system) to obtain compound 4A. SFC detection (ee: 99.66 %), chromatographic column: Chiralpak AS-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.70 min. ¹HNMR (400 MHz, CDCl₃) δ ppm 7.34-7.43 (m, 1H), 6.21-6.35 (m, 2H), 4.26-4.34 (m, 1H), 4.22 (s, 1H), 3.63 (dd, *J*=8.91, 3.76 Hz, 1H), 3.45 (d, *J*=9.16 Hz, 1H), 2.39 (br s, 1H), 1.75-1.93 (m, 2H), 0.73-0.93 (m, 1H), 0.16-0.29 (m, 1H); LCMS (ESI) m/z: 301 [M+1]⁺.

### Embodiment 5

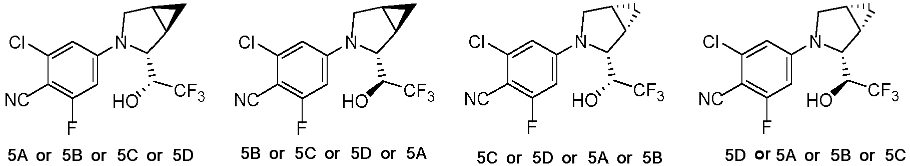

### Synthetic route:

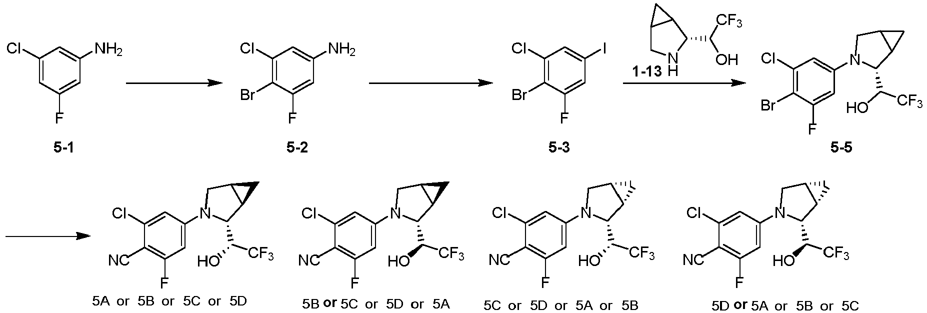

### 1) Synthesis of compound 5-2

Compound 5-1 (2 g, 13.74 mmol) and acetonitrile (10 mL) were added to a pre-dried reaction flask, and the reaction solution was cooled to 0 °C, then a solution of *N-*bromosuccinimide (2.45 g, 13.74 mmol) in acetonitrile (10 mL) was added; the reaction system was replaced with nitrogen for three times, and the reaction was carried out at 0 °C for 2 hours. Water (20 mL) was added to the reaction solution, and the mixture was extracted with ethyl acetate (20 mL^{∗}3); the organic phases were collected, washed with saturated brine (20 mL) in turn, dried over anhydrous sodium sulfate, filtered, and the filtrate was concentrated under reduced pressure to obtain a crude product, and the crude product was purified by column chromatography to obtain compound 5-2. ¹HNMR (400 MHz, CDCl₃) δ ppm 6.62-6.61 (m, 1H), 6.39-6.36 (m, 1H), 3.85 (br s, 2H).

### 2) Synthesis of compound 5-3

Compound 5-2 (1 g, 4.46 mmol) and concentrated hydrochloric acid (12 M, 6 mL) were added to a pre-dried reaction flask, and the mixture was cooled to 0 °C, then a mixed solution of sodium nitrite (338 mg, 4.90 mmol) and water (1.5 mL) was added thereto; and the mixture was stirred at 0 °C for 1 hour, and sodium iodide (734 mg, 4.90 mmol) was added thereto, then the mixture was stirred at 0 °C for 1 hour. The reaction solution was poured into water (10 mL), and the mixture was extracted with ethyl acetate (10 mL^{∗}3); the organic phases were collected, washed with saturated brine (10 mL) in turn, dried over anhydrous sodium sulfate, filtered, and the filtrate was concentrated under reduced pressure to obtain a crude product, and the crude product was purified by column chromatography to obtain compound 5-3. ¹HNMR (400 MHz, CDCl₃) δ ppm 7.63-7.62 (m, 1H), 7.40-7.38 (m, 1H).

### 3) Synthesis of compound 5-5

Compound 5-3 (0.3 g, 894.62 µmol), 1-13 (162.06 mg, 894.62 µmol), cesium carbonate (582 mg, 1.79 mmol), tris(dibenzylideneacetone)dipalladium (81 mg, 89.46 µmol), 2,2-bis(diphenylphosphino)-1,1-binaphthyl (55 mg, 89.46 µmol), and dioxane (4 mL) were added to a pre-dried reaction flask, and the reaction solution was stirred at 110 °C for 5 hours under the protection of nitrogen. The reaction solution was concentrated under reduced pressure to obtain a crude product, and the crude product was purified by preparative thin-layer chromatography on silica gel plates to obtain compound 5-5. LCMS (ESI) m/z: 388 [M+1]⁺.

### 4) Synthesis of compound 5A or 5B or 5C or 5D

Compound 5-5 (35 mg, 90.07 µmol), 1,1-bis(diphenylphosphino)ferrocene (10 mg, 18.01 µmol), *N*,*N*-dimethylformamide (1 mL), zinc cyanide (7 mg, 63.05 µmol) and tris(dibenzylideneacetone)dipalladium (4 mg, 4.50 µmol) were added to a pre-dried reaction flask, and the microwave reaction was carried out at 130 °C for half an hour under the protection of nitrogen. The reaction solution was concentrated under reduced pressure to obtain a crude product, and the crude product was purified by preparative thin-layer chromatography on silica gel plates, purified by preparative high performance liquid chromatography (neutral system), and resolved by supercritical fluid chromatography (alkaline system) in turn to obtain compound 5A. SFC detection (ee: 87.7 %), chromatographic column: Chiralpak AD-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.77 min. ¹HNMR (400 MHz, CDCl₃) δ ppm 6.38 (s, 1H), 6.18 (d, *J*=11.6 Hz, 1H), 4.35-4.29 (m, 1H), 4.17 (s, 1H), 3.68-3.64 (m, 1H), 3.44-3.43 (m, 1H), 2.55 (br s, 1H), 1.87-1.81 (m, 2H), 0.88-0.85 (m, 1H), 0.20-0.18 (m, 1H); LCMS (ESI) m/z: 335 [M+1]⁺.

### Embodiment 6

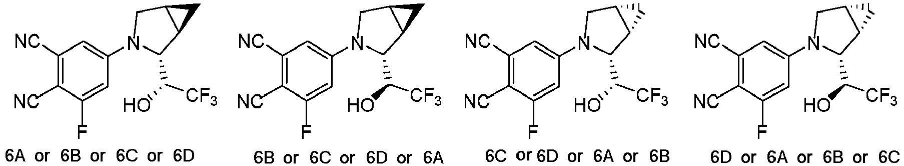

### Synthetic route:

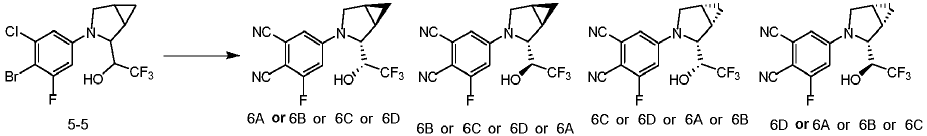

### 4) Synthesis of compound 6A or 6B or 6C or 6D

Compound 5-5 (44 mg, 113.23 µmol), 1,1-bis(diphenylphosphino)ferrocene (12 mg, 22.65 µmol), *N*,*N*-dimethylformamide (1 mL), zinc cyanide (9 mg, 79.26 µmol) and tris(dibenzylideneacetone)dipalladium (5 mg, 5.66 µmol) were added to a pre-dried reaction flask, and the microwave reaction was carried out at 130 °C for half an hour under the protection of nitrogen. The reaction solution was concentrated under reduced pressure to obtain a crude product, and the crude product was purified by preparative thin-layer chromatography on silica gel plates and preparative high performance liquid chromatography (neutral system) in turn to obtain a mixture of compound 6A and compound 6B. SFC detection (the ratio was 49: 51), chromatographic column: Chiralpak AD-3 150 x 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: compound 6A (1.84 min) and compound 6A (2.08 min). ¹HNMR (400 MHz, CDCl₃) δ ppm 6.65 (s, 1H), 6.46 (d, *J*=14.0 Hz, 1H), 4.29-4.21 (m, 2H), 3.72-3.68 (m, 1H), 3.48-3.45 (m, 1H), 2.67 (br s, 1H), 1.90-1.86 (m, 2H), 0.92-0.89 (m, 1H), 0.20-0.18 (m, 1H); LCMS (ESI) m/z: 326 [M+1]⁺.

### Embodiment 7

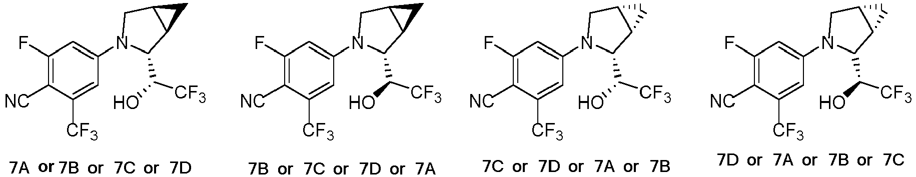

### Synthetic route:

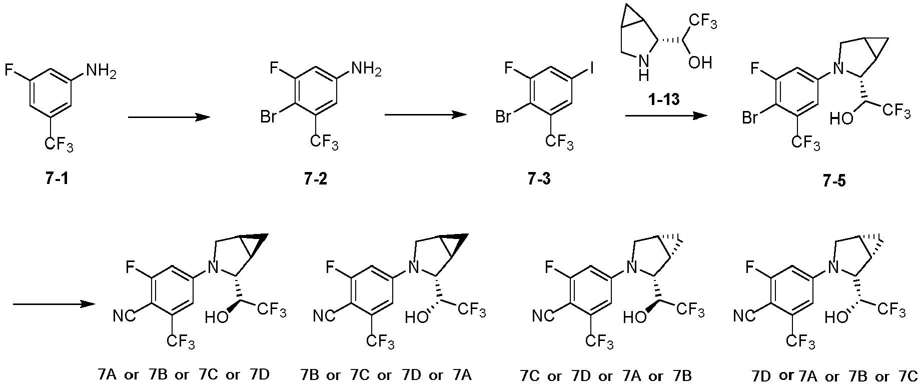

### 1) Synthesis of compound 7-2

Compound 7-1 (3 g, 16.75 mmol), acetonitrile (30 mL) were added to a single-necked flask, and the mixture was cooled to 0 °C, then a solution of *N*-bromosuccinimide (3.00 g, 16.86 mmol) in acetonitrile (30 mL) was added thereto; the reaction system was replaced with nitrogen for three times, and the reaction was carried out at 0 °C for 2 hours. The reaction solution was concentrated under reduced pressure to remove the solvent to obtain a crude product, and the crude product was purified by column chromatography to obtain compound 7-2. ¹HNMR (400 MHz, CDCl₃) δ ppm 6.81 (d, *J*=1.0 Hz, 1H), 6.59 (dd, *J* = 2.4, 9.8 Hz, 1H), 3.99 (br s, 2H).

### 2) Synthesis of compound 7-3

Compound 7-2 (1 g, 3.88 mmol) and concentrated hydrochloric acid (10 mL) were added to a pre-dried three-necked flask, and an aqueous solution (5 mL) of sodium nitrite (294 mg, 4.26 mmol) was added thereto, then the mixture was stirred for 20 min; sodium iodide (639 mg, 4.26 mmol) was added thereto at 0 °C, and the mixture was slowly warmed to 10 °C and stirred for 4 hours and 10 minutes. Ethyl acetate (30 mL) and saturated brine (30 mL) were added to the reaction solution, then the phases were separated; the organic phases were collected, dried over anhydrous sodium sulfate, filtered, and the filtrate was concentrated under reduced pressure to obtain a crude product, and the crude product was purified by column chromatography to obtain compound 7-3. ¹HNMR (400 MHz, CDCl₃) δ ppm 7.81 (s, 1H), 7.66 (dd, *J*=1.8, 7.0 Hz, 1H).

### 3) Synthesis of compound 7-5

Compound 1-13 (100 mg, 552.01 µmol), compound 7-3 (203 mg, 552.01 µmol), 2,2-bis(diphenylphosphino)-1,1-binaphthyl (34 mg, 55.20 µmol), tris(dibenzylideneacetone) dipalladium (50.55 mg, 55.20 µmol) and cesium carbonate (449 mg, 1.38 mmol) were added to a pre-dried single-necked flask, then 1,4-dioxane (3 mL) was added thereto, and the reaction system was evacuated and replaced with nitrogen for three times, then the mixture was stirred in an oil bath at 100 °C for 32 hours. The reaction solution was concentrated under reduced pressure to remove the solvent to obtain a crude product, and the crude product was purified by preparative thin-layer chromatography on silica gel plates and preparative high performance liquid chromatography (neutral system) to obtain compound 7-5. ¹HNMR (400 MHz, CDCl₃) δ ppm 6.66 (d, *J*=2.2 Hz, 1H), 6.43 (dd, *J*=2.6, 11.0 Hz, 1H), 4.32-4.21 (m, 1H), 4.20 (s, 1H), 3.60 (dd, *J*=3.5, 8.8 Hz, 1H), 3.40 (d, *J*=8.8 Hz, 1H), 2.24 (br d, *J*=6.6 Hz, 1H), 1.89-1.73 (m, 2H), 0.83 (dt, *J*=5.3, 7.7 Hz, 1H), 0.24 (q, *J*=4.4 Hz, 1H).

### 4) Synthesis of compound 7A or 7B or 7C or 7D

Compound 7-5 (20 mg, 47.38 µmol), zinc cyanide (6 mg, 56.85 µmol), tetrakis(triphenylphosphine)palladium (8 mg, 7.11 µmol), and *N*-methylpyrrolidone (1 mL) were added to a pre-dried microwave tube, and the reaction system was blown with nitrogen for 1 min and then sealed, then the mixture was stirred at 130 °C for 0.5 hours in the microwave. 5 mL of saturated brine and 5 mL of ethyl acetate were added to the reaction solution, and the phases were separated; the organic phase was collected, dried over anhydrous sodium sulfate, filtered, and the filtrate was concentrated under reduced pressure to obtain a crude product, and the crude product was purified by preparative thin-layer chromatography on silica gel plates and resolved by supercritical fluid chromatography (alkaline system) to obtain compound 7A. SFC detection (ee: 98.76 %), chromatographic column: Chiralpak AD-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.00 min. ¹HNMR (400 MHz, CDCl₃) δ ppm 6.65 (d, *J*=1.5 Hz, 1H), 6.38 (s, 1H), 4.29-4.24 (m, 2H), 3.69 (dd, *J*=4.0, 9.1 Hz, 1H), 3.50 (br d, *J*=9.0 Hz, 1H), 2.42 (s, 1H), 1.93-1.78 (m, 2H), 0.92-0.85 (m, 1H), 0.25-0.16 (m, 1H); LCMS (ESI) m/z: 369 [M+1]⁺.

### Embodiment 8

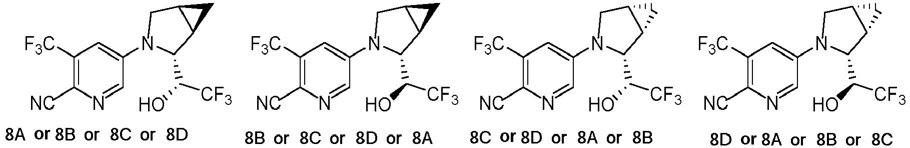

### Synthetic route:

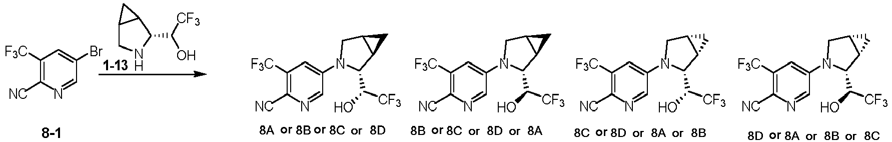

### 1) Synthesis of compound 8A or 8B or 8C or 8D

Compound 1-13 (30 mg, 165.60 µmol), compound 8-1 (41 mg, 165.60 µmol), 2,2-bis(diphenylphosphino)-1,1-binaphthyl (10 mg, 16.56 µmol), tris(dibenzylideneacetone) dipalladium (15 mg, 16.56 µmol) and cesium carbonate (87 mg, 414.01µmol) were added to a pre-dried single-necked flask, then 1,4-dioxane (1 mL) was added thereto, and the reaction system was evacuated and replaced with nitrogen for three times, then the mixture was stirred at 100 °C for 9 hours. The reaction solution was concentrated under reduced pressure to obtain a crude product, and the crude product was purified by preparative thin-layer chromatography on silica gel plates, purified by preparative high performance liquid chromatography (alkaline system), and resolved by supercritical fluid chromatography (alkaline system) in turn to obtain compound 8A. SFC detection (ee: 99.8 %), chromatographic column: Chiralpak AD-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.18 min. ¹HNMR (400 MHz, CDCl₃) δ ppm 8.14 (d, *J*=2.9 Hz, 1H), 7.02 (d, *J*=2.7 Hz, 1H), 4.40-4.33 (m, 1H), 4.29-4.18 (m, 1H), 3.74 (dd, J=3.9, 9.4 Hz, 1H), 3.56 (d, *J*=9.2 Hz, 1H), 2.64-2.52 (m, 1H), 2.04-1.76 (m, 2H), 0.91 (dt, *J*=5.5, 7.8 Hz, 1H), 0.22 (q, *J*=4.4 Hz, 1H); LCMS (ESI) m/z: 352 [M+1]⁺.

### Embodiment 9

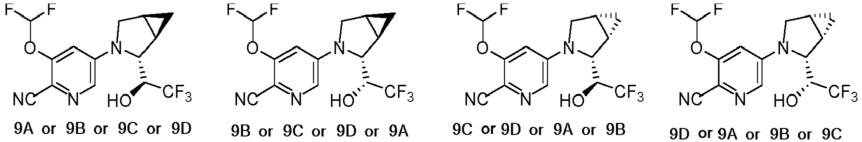

### Synthetic route:

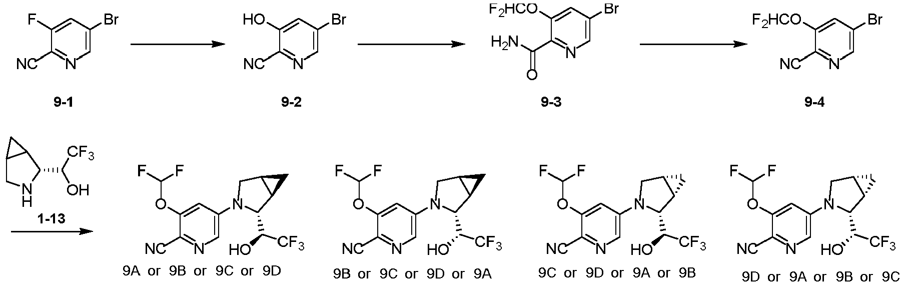

### 1) Synthesis of compound 9-2

Compound 9-1 (1.5 g, 7.46 mmol), tetrahydrofuran (20 mL), and 2-methylsulfonyl ethanol (1.39 g, 11.19 mmol) were added to a three-necked flask, and the mixture was cooled to 0 °C, then sodium hydride (895 mg, 22.39 mmol) with 60 % purity was added thereto; the mixture was stirred at 0 °C for 1 hour and then naturally warmed to 25 °C and then stirred for 16 hours. The reaction solution was quenched with saturated ammonium chloride (30 mL), extracted with ethyl acetate (30 mL^{∗}3); the organic phases were combined, washed with saturated brine (20 mL^{∗}3), dried over anhydrous sodium sulfate, filtered, and the filtrate was concentrated under reduced pressure to obtain a crude product, and the crude product was purified by column chromatography to obtain compound 9-2. ¹HNMR (400 MHz, MeOD) δ ppm 8.08 (d, *J*=1.76 Hz, 1H), 7.50 (d, *J*=1.88 Hz, 1H).

### 2) Synthesis of compound 9-3

Compound 9-2 (800 mg, 4.02 mmol), potassium hydroxide (1.24 g, 22.11 mmol), difluoromethyl phenyl sulfone (1.55 g, 8.04 mmol, 1.14 mL), acetonitrile (5 mL), and water (1.5 mL) were added to a thumb bottle, and the reaction was carried out at 70 °C for 4 hours. The reaction solution was concentrated under reduced pressure to obtain a crude product, and the crude product was purified by column chromatography to obtain compound 9-3. ¹HNMR (400 MHz, CDCl₃) δ ppm 8.56 (d, *J*=1.96 Hz, 1H), 7.88 (d, *J*=0.78 Hz, 1H), 6.61-7.03 (m, 1H).

### 3) Synthesis of compound 9-4

Compound 9-3 (200 mg, 748.99 µmol), dichloromethane (2 mL), triethylamine (379 mg, 3.74 mmol, 521.25 µL) were added to a three-necked flask, and the mixture was cooled to 0 °C, then trifluoroacetic anhydride (314.62 mg, 1.50 mmol, 208.36 µL) was added thereto; the reaction was carried out at 0 °C for 1 hour, naturally warmed to 25 °C and then carried out for 2 hours. Water (5 mL) was added to the reaction solution, then the mixture was extracted with dichloromethane (5 mL); the organic phases were collected, dried over anhydrous sodium sulfate, filtered, and the filtrate was concentrated under reduced pressure to obtain a crude product, and the crude product was purified by column chromatography to obtain compound 9-4. ¹HNMR (400 MHz, CDCl₃) δ ppm 8.65 (d, *J*=1.76 Hz, 1H), 7.94 (s, 1H), 6.46-6.98 (m, 1H).

### 4) Synthesis of compound 9A or 9B or 9C or 9D

Compound 1-13 (50 mg, 276.01 µmol), compound 9-4 (68 mg, 276.01 µmol), potassium phosphate (175 mg, 828.02 µmol), tris(dibenzylideneacetone)dipalladium (25 mg, 27.60 µmol), 2,2-bis(diphenylphosphino)-1,1-binaphthyl (17 mg, 27.60 µmol), and dioxane (2 mL) were added to a thumb bottle, and the reaction system was replaced with nitrogen for three times, then the reaction was carried out at 100 °C for 4 hours. The reaction solution was concentrated under reduced pressure to obtain a crude product, and the crude product was purified by column chromatography and resolved by supercritical fluid chromatography to obtain compound 9A. SFC detection (ee: 100 %), chromatographic column: Lµx Cellµlose-2 50 × 4.6 mm I.D., 3 µm; mobile phase: A: supercritical carbon dioxide, B: 0.05 % isopropanol solution of isopropylamine; gradient: from 5 % to 40 % in 1 minute, held 40 % for 1 min, returned to 5 % in 0.5 min, equilibrated at 5 % for 1.5 min; flow rate: 4 mL/min; column temperature: 35 °C; wavelength: 220 nm, retention time: 1.39 min. ¹HNMR (400 MHz, CDCl₃) δ ppm 7.88 (d, *J*=2.38 Hz, 1H), 6.45-6.87 (m, 2H), 4.19-4.36 (m, 2H), 3.70 (dd, *J*=9.03, 3.76 Hz, 1H), 3.51 (d, *J*=9.03 Hz, 1H), 2.49 (d, *J*=6.90 Hz, 1H), 1.88 (dt, *J*=8.44, 4.38 Hz, 2H), 0.81-0.94 (m, 1H), 0.18-0.31 (m, 1H); LCMS (ESI) m/z: 350 [M+1]⁺.

### Embodiment 10

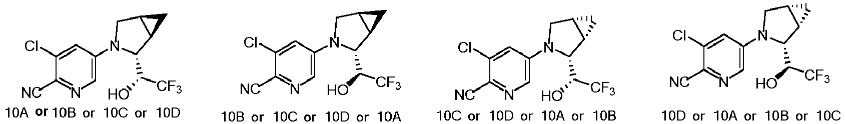

### Synthetic route:

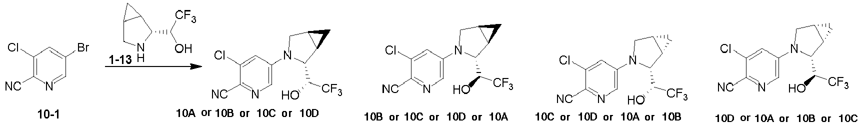

### 1) Synthesis of compound 10A or 10B or 10C or 10D

Compound 1-13 (30 mg, 165.60 µmol), compound 10-1 (36 mg, 165.60 µmol), 2,2-bis(diphenylphosphino)-1,1-binaphthyl (10 mg, 16.56 µmol), tris(dibenzylideneacetone) dipalladium (15 mg, 16.56 µmol) and potassium phosphate (87 mg, 414.00 µmol) were added to a pre-dried single-necked flask, then 1,4-dioxane (1 mL) was added thereto, and the reaction system was evacuated and replaced with nitrogen for three times, then the mixture was stirred at 100 °C for 16 hours. The reaction solution was concentrated under reduced pressure to remove the solvent to obtain a crude product, and the crude product was purified by preparative thin-layer chromatography on silica gel plates and resolved by supercritical fluid chromatography (alkaline system) in turn to obtain compound 10A. SFC detection (ee: 100 %), chromatographic column: Lµx Cellµlose-2 150^{∗}4.6 mm 3 µm; mobile phase: A: supercritical carbon dioxide, B: 0.05 % ethanol solution of diethylamine; gradient: from 5 % to 40 % of B in 5.5 minute, held 40 % for 3.0 min, equilibrated at 5 % for 1.5 min; flow rate: 2.5 mL/min; column temperature: 40 °C; wavelength: 280 nm, retention time: 3.24 min. ¹HNMR (400 MHz, CDCl₃) δ ppm 7.89 (d, *J*=2.7 Hz, 1H), 6.81 (d, *J*=2.5 Hz, 1H), 4.31-4.25 (m, 2H), 3.71 (dd, *J*=3.8, 9.1 Hz, 1H), 3.49 (d, *J*=9.4 Hz, 1H), 3.06-2.52 (m, 1H), 1.87 (ddd, J=3.9, 7.7, 11.7 Hz, 2H), 0.88 (dt, *J*=5.6, 7.8 Hz, 1H), 0.24-0.17 (m, 1H); LCMS (ESI) m/z: 318 [M+1]⁺.

### Embodiment 11

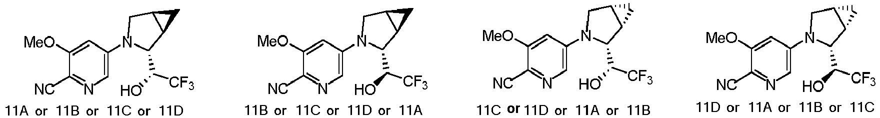

### Synthetic route:

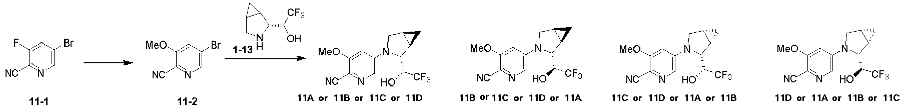

### 1) Synthesis of compound 11-2

Compound 11-1 (0.5 g, 2.49 mmol), sodium methoxide (671 mg, 12.44 mmol), and tetrahydrofuran (5 mL) were added to a thumb bottle, and the reaction system was replaced with nitrogen for three times, then the reaction was carried out at 25 °C for 1 hour. The reaction solution was concentrated under reduced pressure to obtain a crude product, and the crude product was purified by column chromatography to obtain compound 11-2. ¹HNMR (400 MHz, CDCl₃) δ ppm 8.35 (d, *J*=1.56 Hz, 1H), 7.53 (d, *J*=1.76 Hz, 1H), 4.00 (s, 3H).

### 2) Synthesis of compound 11A or 11B or 11C or 11D

Compound 1-13 (100 mg, 552.01 µmol), compound 11-2 (117 mg, 552.01 µmol), potassium phosphate (351 mg, 1.66 mmol), tris(dibenzylideneacetone)dipalladium (51 mg, 55.20µmol), 2,2-bis(diphenylphosphino)-1,1-binaphthyl (34 mg, 55.20µmol), and dioxane (2 mL) were added to a thumb bottle, and the reaction system was replaced with nitrogen for three times, then the reaction was carried out at 100 °C for 16 hours. The reaction solution was concentrated under reduced pressure to obtain a crude product, then the crude product was purified by column chromatography, purified by preparative high performance liquid chromatography (alkaline system) and resolved by supercritical fluid chromatography (alkaline system) in turn to obtain compound 11A. SFC detection (ee: 97.62 %), chromatographic column: Chiralpak OD-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 2.08 min. ¹HNMR (400 MHz, CDCl₃) δ ppm 7.56 (d, J=2.26 Hz, 1H), 6.25 (d, *J*=2.13 Hz, 1H), 4.24-4.40 (m, 2H), 3.91 (s, 3H), 3.73 (dd, *J*=9.10, 3.70 Hz, 1H), 3.49 (d, *J*=9.29 Hz, 1H), 3.04 (br s, 1H), 1.86 (br dd, *J*=7.84, 3.83 Hz, 2H), 0.80-0.94 (m, 1H), 0.16-0.28 (m, 1H); LCMS (ESI) m/z: 314 [M+1]⁺.

### Embodiment 12

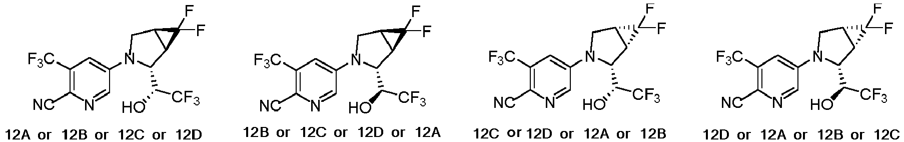

### Synthetic route:

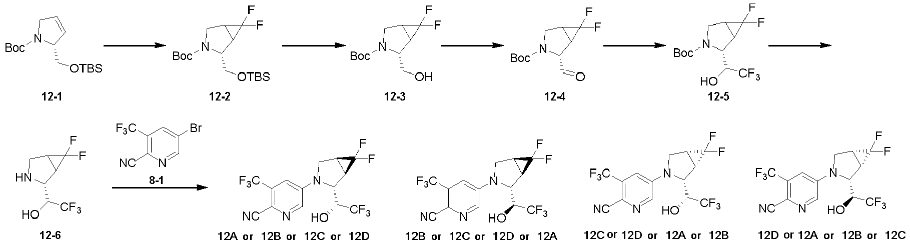

### 1) Synthesis of compound 12-2

Compound 12-1 (1.5 g, 4.78 mmol), sodium iodide (717.16 mg, 4.78 mmol), trifluoromethyl trimethylsilane (4.08 g, 28.71 mmol), and tetrahydrofuran (20 mL) were added to a round bottom flask, and the reaction system was replaced with nitrogen for three times, then the reaction was carried out at 110 °C for 48 hours. The reaction solution was concentrated under reduced pressure to obtain a crude product, and the crude product was purified by column chromatography to obtain compound 12-2.

### 2) Synthesis of compound 12-3

Compound 12-2 (3 g, 8.25 mmol), tetrabutylammonium fluoride (1 M, 9.90 mL), and tetrahydrofuran (30 mL) were added to a round bottom flask, and the reaction system was replaced with nitrogen for three times, then the reaction was carried out at 25 °C for 2 hours. Ethyl acetate (30 mL) was added to the reaction solution, and the organic phase was washed with 1 M hydrochloric acid aqueous solution (30 mL^{∗}3), dried over anhydrous sodium sulfate, filtered, and the filtrate was concentrated under reduced pressure to obtain a crude product, and the crude product was purified by column chromatography to obtain compound 12-3. ¹HNMR (400 MHz, CDCl₃) δ ppm 3.94-4.40 (m, 2H), 3.69-3.90 (m, 3H), 3.61 (dt, *J*=10.45, 5.17 Hz, 1H), 2.03-2.30 (m, 2H), 1.46 (s, 9H); LCMS (ESI) m/z: 193 [M-55]⁺.

### 3) Synthesis of compound 12-4

Compound 12-3 (460 mg, 1.85 mmol), *N*,*N*-diisopropylethylamine (1.43 g, 11.07 mmol, 1.93 mL), ethyl acetate (5 mL) were added to a three-necked flask, and the reaction system was replaced with nitrogen for three times, then the mixture was cooled to 0 °C; a solution of pyridine sulfur trioxide (881.19 mg, 5.54 mmol) in dimethyl sulfoxide (5 mL) was added thereto, and then the mixture was stirred at 0 °C for 2 hours. Ethyl acetate (10 mL) was added to the reaction solution, and the organic phase was washed with saturated brine solution (10 mL^{∗}3), and then washed with 1 M hydrochloric acid (10 mL^{∗}3); the organic phase was dried over anhydrous sodium sulfate, filtered, and the filtrate was concentrated under reduced pressure to obtain crude compound 12-4. ¹HNMR (400 MHz, CDCl₃) δ ppm 9.56-9.71 (m, 1H), 3.51-3.85 (m, 3H), 2.29-2.49 (m, 2H), 1.43-1.46 (m, 9H).

### 4) Synthesis of compound 12-5

Compound 12-4 (0.4 g, 1.62 mmol), (trifluoromethyl)trimethylsilane (345.08 mg, 2.43 mmol), tetrahydrofuran (4 mL) were added to a three-necked flask, and the reaction system was replaced with nitrogen for three times, then the mixture was cooled to 0 °C; tetrabutylammonium fluoride (1 M, 3.24 mL) was added thereto, and the mixture was stirred at 0 °C for 2 hours. Ethyl acetate (10 mL) was added to the reaction solution, and the organic phase was washed with 1 M hydrochloric acid (10 mL^{∗}3), then the organic phase was collected, dried over anhydrous sodium sulfate, filtered, then the filtrate was concentrated under reduced pressure to obtain a crude product, and the crude product was purified by column chromatography to obtain compound 12-5. LCMS (ESI) m/z: 262 [M-55]⁺.

### 5) Synthesis of compound 12-6

Compound 12-5 (200 mg, 630.41 µmol), hydrochloric acid/ethyl acetate (6 M, 10 mL) were added to a three-necked flask, and the reaction system was replaced with nitrogen for three times, then the reaction was carried out at 0 °C for 2 hours. Water (5 mL) was added to the reaction system, and the phases were separated, then the aqueous phase was collected; the pH of the aqueous phase was adjusted to 11 with saturated potassium carbonate solution, and the mixture was extracted with ethyl acetate (10 mL^{∗}3), then the organic phases were combined, washed with saturated brine (5 mL^{∗}3), dried over anhydrous sodium sulfate, filtered, and the filtrate was concentrated under reduced pressure to obtain crude compound 12-6. ¹HNMR (400 MHz, MeOD) δ ppm 4.85 (s, 1 H), 4.49-4.66 (m, 1 H), 4.14-4.28 (m, 1 H), 3.74-3.90 (m, 1 H), 3.58 (t, *J*=10.96 Hz, 1 H), 3.30-3.32 (m, 1 H), 2.74-2.98 (m, 2 H); LCMS (ESI) m/z: 217 [M+1]⁺.

### 6) Synthesis of compound 12A or 12B or 12C or 12D

Compound 12-6 (115.60 mg, 60.54 µmol), compound 8-1 (100 mg, 460.54 µmol), methanesulfonato(2-dicyclohexylphosphino-3,6-dimethoxy-2',4',6'-tri-i-propyl-1,1'-biphenyl)(2'-amino-1,1'-biphenyl-2-yl)palladium (II) (41 mg), tetrahydrofuran (9 mL), sodium *tert*-butoxide (88.5 mg, 921.08 µmol) were added to a reaction flask under nitrogen atmosphere, and the mixture was stirred at 100 °C for 18 hours. The reaction solution was concentrated under reduced pressure to obtain a crude product, and the crude product was purified by preparative thin-layer chromatography on silica gel plates and preparative high performance liquid chromatography (neutral system) in turn to obtain a mixture of compound 12A and compound 12B. SFC detection (the ratio was 50: 50), chromatographic column: Chiralpak AD-3 150 × 4.6 mm I.D., 3 µm; mobile phase: A: supercritical carbon dioxide, B: 0.05 % isopropanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: compound 12A(0.89 min) and compound 12B (1.06 min). ¹HNMR (400 MHz, CDCl₃) δ ppm 8.77 (d, *J*=2.64 Hz, 1H), 7.92 (d, *J*=2.64 Hz, 1H), 7.27 (s, 1H), 4.73-4.84 (m, 1H), 4.07 (br s, 1H), 3.31 (br d, *J*=11.80 Hz, 1H), 2.84-2.97 (m, 1H), 2.50 (dd, *J*=13.80, 8.66 Hz, 1H), 2.22-2.41 (m, 2H); LCMS (ESI) m/z: 388 [M+1]⁺.

### Embodiment 13

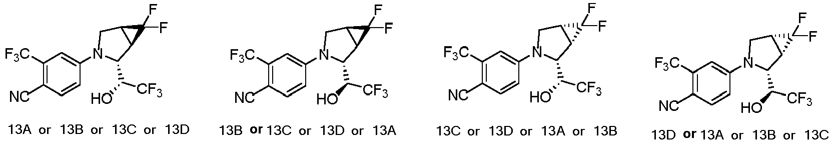

### Synthetic route:

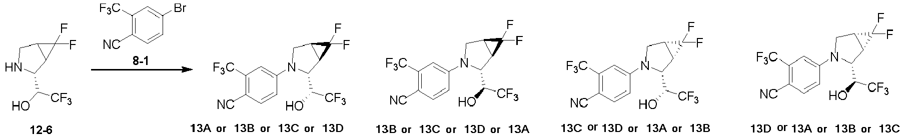

### 1) Synthesis of compound 13A or 13B or 13C or 13D

Compound 12-6 (70 mg, 322.38 µmol), compound 8-1 (88.66 mg, 354.62 µmol), cesium carbonate (262.59 mg, 805.95 µmol), tris(dibenzylideneacetone)dipalladium (29.52 mg, 32.24 µmol), 2,2-bis(diphenylphosphino)-1,1-binaphthyl (20.07 mg, 32.24 µmol), and dioxane (1 mL) were added to a thumb bottle, and the reaction system was replaced with nitrogen for three times, then the reaction was carried out at 100 °C for 3 hours. The reaction solution was concentrated under reduced pressure to obtain a crude product, and the crude product was purified by preparative thin-layer chromatography on silica gel plates, purified by preparative high performance liquid chromatography (acidic system), and resolved by supercritical fluid chromatography (alkaline system) in turn to obtain compound 13A. SFC detection (ee: 98.66 %), chromatographic column: Chiralpak AS-3 150 × 4.6 mm I.D., 3 µm; mobile phase: A: supercritical carbon dioxide, B: 0.05 % isopropanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.16 min. ¹HNMR (400 MHz, CDCl₃) δ ppm 7.61 (d, *J*=8.91 Hz, 1H), 6.83 (d, *J*=2.13 Hz, 1H), 6.66 (dd, *J*=8.85, 2.70 Hz, 1H), 4.52 (s, 1H) 4.29-4.42 (m, 1 H), 3.86-3.97 (m, 1H), 3.76 (d, *J*=9.79 Hz, 1H), 2.49-2.75 (m, 3H), 1.55 (s, 4H); LCMS (ESI) m/z: 386 [M+1]⁺.

### Embodiment 14

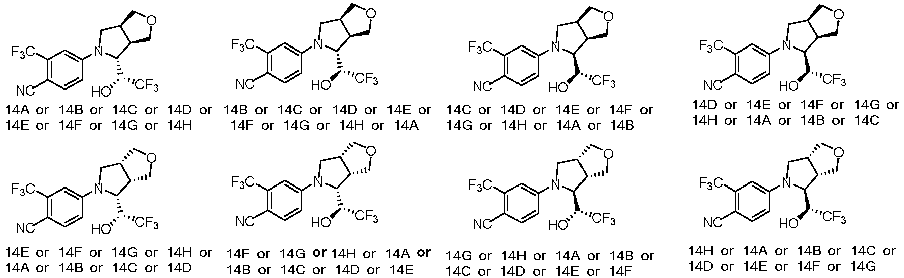

### Synthetic route:

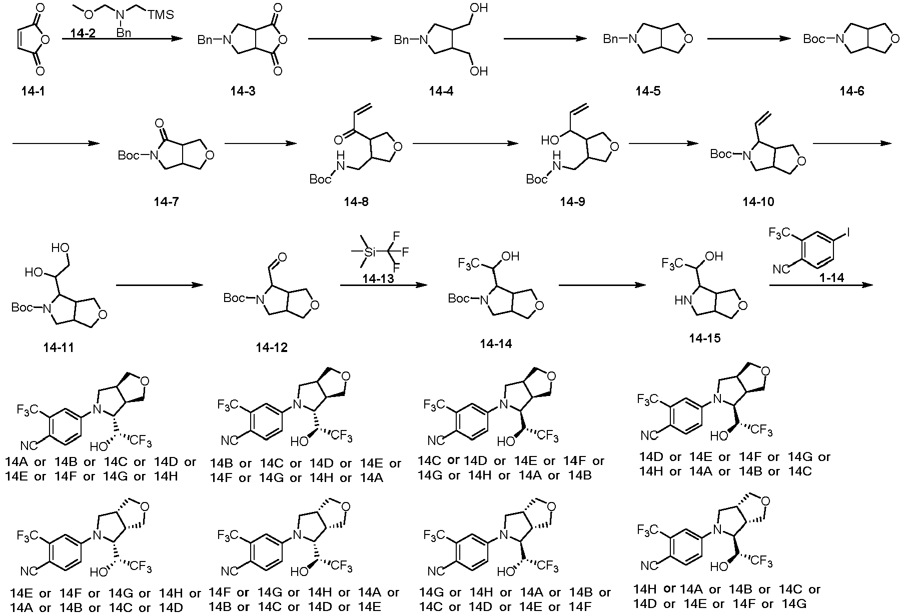

### 1) Synthesis of compound 14-3

Compound 14-1 (50 g, 509.91 mmol), dichloromethane (450 mL), and compound 14-2 (122.27 g, 515.01 mmol) were added to a pre-dried three-necked flask, and a mixed solution of dichloromethane (50 mL) and trifluoroacetic acid (5.81 g, 50.99 mmol) was added dropwise thereto in an ice bath at a controlled temperature of 0 °C; the temperature was controlled not to exceed 30 °C, and after the dropwise addition was completed, the mixture was stirred at 30 °C for 3 hours. 500 mL of water was added to the reaction solution, and the phases were separated, then the organic phase was collected, dried over anhydrous sodium sulfate, filtered, and the filtrate was concentrated under reduced pressure to obtain crude compound 14-3.

### 2) Synthesis of compound 14-4

Compound 14-3 (117.9 g, 509.85 mmol) and tetrahydrofuran (1.3 L) were added to a pre-dried three-necked flask, then lithium aluminum hydride (38.70 g, 1.02 mol) was added thereto, and the mixture was stirred at 25 °C for 2 hours. The reaction was quenched by adding water (40 mL), 15 % sodium hydroxide aqueous solution (40 mL) and water (120 mL) dropwise in turn to the reaction solution in an ice bath. The mixture was filtered with diatomite, and the filter cake was rinsed with ethyl acetate (500 mL^{∗}5), then the filtrate was concentrated under reduced pressure to about 300 mL; saturated brine (100 mL) was added thereto, and the phases were separated, then the organic phase was collected, dried over anhydrous sodium sulfate, filtered, and the filtrate was concentrated under reduced pressure to obtain crude compound 14-4. ¹HNMR (400 MHz, CDCl₃) δ ppm 7.61 - 7.06 (m, 5H), 3.75 - 3.70 (m, 3H), 3.57 (s, 2H), 2.61 (d, *J* = 5.9 Hz, 3H), 2.51 - 2.44 (m, 1H), 2.18 - 1.97 (m, 1H).

### 3) Synthesis of compound 14-5

Compound 14-4 (80 g, 361.5 mmol), potassium *tert*-butoxide (121.70 g, 1.08 mol), tetrahydrofuran (1000 mL) were added to a pre-dried three-necked flask, then *p-*toluenesulfonyl chloride (72.37 g, 379.58 mmol) was added in batches at 0 °C in an ice bath; the temperature was controlled not to exceed 50 °C, and after the addition was completed, the reaction was heated to 66 °C and carried out for 16 hours. The pH of the reaction solution was adjusted to 8 with concentrated hydrochloric acid, then saturated brine (500 mL) and ethyl acetate (300 mL^{∗}2) were added, and the phases were separated; the organic phases were collected, dried over anhydrous sodium sulfate, filtered, and the filtrate was concentrated under reduced pressure to obtain a crude product, and the crude product was purified by column chromatography to obtain compound 14-5. ¹HNMR (400 MHz, CDCl₃) δ ppm 7.35 - 6.89 (m, 5H), 3.74 - 3.66 (m, 2H), 3.53 - 3.42 (m, 4H), 2.78 - 2.66 (m, 2H), 2.62 - 2.49 (m, 2H), 2.34 - 2.17 (m, 2H).

### 4) Synthesis of compound 14-6

Compound 14-5 (25 g, 122.98 mmol), ethyl acetate (500 mL), wet palladium carbon (25 g) with 10 % purity were added to a pre-dried hydrogenation bottle, then Boc anhydride (53.68 g, 245.97 mmol, 56.51 mL) was added thereto, and hydrogen was introduced into the reaction system, and the mixture was stirred at 50 °C-50 psi for 3 hours. The reaction solution was filtered, and the filtrate was concentrated under reduced pressure to obtain a crude product; ethyl acetate (100 mL) and *N*,*N*-dimethylethylenediamine (15 mL) were added to the crude product, and the mixture was stirred at 30 °C for 3 hours, then the pH of the mixture was adjusted to 1-2 with 1 N dilute hydrochloric acid, and the phases were separated; the organic phase was dried over anhydrous sodium sulfate, filtered, and the filtrate was concentrated under reduced pressure to obtain crude compound 14-6. ¹HNMR (400 MHz, CDCl₃) δ ppm 3.91-3.74 (m, 2H), 3.65-3.45 (m, 4H), 3.37-3.10 (m, 2H), 2.97-2.76 (m, 2H), 1.44-1.39 (m, 9H).

### 5) Synthesis of compound 14-7

Compound 14-6 (17 g, 79.71 mmol) and ethyl acetate (500 mL) were added to a pre-dried single-necked flask, and a mixed solution of water (500 mL) and anhydrous ruthenium trichloride (1.65 g, 7.97 mmol) was slowly added to the reaction solution in an ice-water bath at 10 °C, then sodium periodate (68.20 g, 318.84 mmol) was added thereto, and the mixture was stirred at 30 °C for 2 hours. The reaction was quenched by adding isopropanol (170 mL) to the reaction solution, and the reaction solution turned black. The reaction solution was filtered with diatomite, and the filter cake was washed with ethyl acetate, then the phases were separated; the organic phase was collected, dried over anhydrous sodium sulfate, filtered, and the filtrate was concentrated under reduced pressure to obtain a crude product, and the crude product was purified by column chromatography to obtain compound 14-7. ¹HNMR (400 MHz, CDCl₃) δ ppm 4.26 (dd, *J*=2.1, 9.3 Hz, 1H), 3.95 (dd, *J*=8.9, 11.3 Hz, 1H), 3.86-3.74 (m, 3H), 3.57 (dd, *J*=3.3, 11.4 Hz, 1H), 3.23 (ddd, *J*=2.1, 7.6, 9.4 Hz, 1H), 2.93 (tdd, *J*=3.0, 6.1, 9.2 Hz, 1H), 1.59-1.44 (m, 9H).

### 6) Synthesis of compound 14-8

Compound 14-7 (9.98 g, 43.92 mmol), tetrahydrofuran (200 mL) were added to a three-necked flask, and the reaction solution was cooled to -40 °C, then the reaction system was evacuated and replaced with nitrogen for three times; vinyl Grignard reagent (1 M, 43.92 mL) was added dropwise thereto, and the reaction system was evacuated and replaced with nitrogen for three times, and the reaction was carried out at -40 °C for 2 hours. The reaction was quenched by slowly adding water (100 mL) to the reaction system, then saturated brine (50 mL) was added thereto, and the phases were separated; the aqueous phase was extracted with ethyl acetate (3^{∗}100 mL), and the phases were separated; the organic phases of tetrahydrofuran and ethyl acetate were combined, and dried over anhydrous sodium sulfate, filtered, and the filtrate was concentrated under reduced pressure to obtain a crude product, then the crude product was purified by column chromatography to obtain compound 14-8. ¹HNMR (400 MHz, CDCl₃) δ ppm 6.56-6.28 (m, 2H), 5.91 (d, *J*=10.5 Hz, 1H), 4.38-2.69 (m, 13H), 1.50-1.36 (m, 9H); LCMS (ESI)m/z: 182 [M+1]⁺.

### 7) Synthesis of compound 14-9

Compound 14-8 (8.28 g, 32.43 mmol) and methanol (90 mL) were added to a pre-dried single-necked flask, then cerium (III) chloride heptahydrate (12.08 g, 32.43 mmol) was added thereto in an ice bath at 0 °C; sodium borohydride (2.45 g, 64.86 mmol) was added in batches, and the mixture was stirred at 0 °C for 1 hour. The reaction was quenched with 1 N dilute hydrochloric acid (50 mL) and then extracted with ethyl acetate (50 mL^{∗}3); the organic phases were combined, washed with saturated brine (20 mL^{∗}3), dried over anhydrous sodium sulfate, filtered, and the filtrate was concentrated under reduced pressure to obtain a crude product, and the crude product was purified by column chromatography to obtain compound 14-9. ¹HNMR (400 MHz, CDCl₃) δ ppm 6.04-5.70 (m, 1H), 5.42-5.10 (m, 2H), 4.17-4.07 (m, 1H), 3.96-3.80 (m, 3H), 3.79-3.65 (m, 2H), 3.53-3.44 (m, 1H), 3.37-3.10 (m, 2H), 2.69-2.21 (m, 2H), 1.54-1.37 (m, 9H).

### 8) Synthesis of compound 14-10

Compound 14-9 (130 mg, 505.20 µmol), tetrahydrofuran (5 mL) were added to a pre-dried single-necked flask, then *p*-toluenesulfonyl chloride (105 mg, 555.72 µmol) and potassium *tert*-butoxide (170 mg, 1.52 mmol) were added thereto, and the mixture was stirred at 25 °C for 1 hour. The reaction solution was added with 1 N dilute hydrochloric acid (5 mL), and extracted by adding ethyl acetate (5 mL), and the phases were separated; the organic phase was dried over anhydrous sodium sulfate, filtered, and the filtrate was concentrated under reduced pressure to obtain a crude product, and the crude product was purified by column chromatography to obtain compound 14-10. ¹HNMR (400 MHz, CDCl₃) δ ppm 5.88-5.56 (m, 1H), 5.33-4.98 (m, 1H), 4.11-3.33 (m, 6H), 3.22-2.55 (m, 2H), 1.42 (d, *J*=2.0 Hz, 9H); MS (ESI) m/z: 184 [M-55]⁺.

### 9) Synthesis of compound 14-11

Compound 14-10 (100 mg, 417.87 µmol) and acetone (1 mL) were added to a pre-dried single-necked flask, then osmium tetroxide (2 mg, 8.36 µmol) and *N*-methyl morpholine (88 mg, 752.16 µmol) were added thereto, and the mixture was stirred at 30 °C for 16 hours. Saturated sodium sulfite aqueous solution (5 mL) was added to the reaction solution, and the mixture was stirred for 1 hour. The reaction solution was extracted with dichloromethane (10 mL^{∗}3), then the phases were separated, and the organic phase was collected, dried over anhydrous sodium sulfate, filtered, and the filtrate was concentrated under reduced pressure to obtain a crude product, then the crude product was purified by column chromatography to obtain compound 14-11. ¹HNMR (400 MHz, CDCl₃) δ ppm 4.32-3.32 (m, 10H), 2.96 (s, 3H), 1.53-1.45 (m, 9H).

### 10) Synthesis of compound 14-12

Compound 14-11 (73 mg, 267.08 µmol) and tetrahydrofuran (1.3 mL), water (0.4 mL) were added to a pre-dried single-necked flask, then sodium periodate (102 mg, 480.75 µmol) was added thereto, and the mixture was stirred at 30 °C for 16 hours. Ethyl acetate (10 mL) and water (10 mL) were added to the reaction solution, and the phases were separated, then the organic phase was collected, dried over anhydrous sodium sulfate, filtered, and the filtrate was concentrated under reduced pressure to obtain crude compound 14-12. ¹HNMR (400 MHz, CDCl₃) δ ppm 9.66-9.21 (m, 1H), 4.39-2.72 (m, 9H), 1.46-1.21 (m, 9H).

### 11) Synthesis of compound 14-14

Compound 14-12 (50 mg, 207.23 µmol), tetrahydrofuran (1 mL) and compound 14-13 (35 mg, 248.67 µmol) were added to a pre-dried single-necked flask, then a solution of tetrabutylammonium fluoride in tetrahydrofuran (1 M, 310.84 µL) was added thereto at 0 °C in an ice bath, and the mixture was stirred at 30 °C for 4 hours. Compound 14-13 (70.72 mg, 497.34 µmol) and a solution of tetrabutylammonium fluoride in tetrahydrofuran (1 M, 621.68 µL) were added to the mixture at 0 °C in an ice bath, and the mixture was stirred at 30 °C for 48 hours. Saturated brine (5 mL) was added to the reaction solution for washing, and the phases were separated; the organic phase was collected, washed with 1 N dilute hydrochloric acid (5 mL^{∗}2), and then the phases were separated; the organic phase was dried over anhydrous sodium sulfate, filtered, and the filtrate was concentrated under reduced pressure to obtain a crude product, and the crude product was purified by column chromatography to obtain compound 14-14. ¹HNMR (400 MHz, CDCl₃) δ ppm 4.40-2.71 (m, 10H), 1.57-1.41 (m, 9H); LCMS (ESI) m/z: 256 [M-55]⁺.

### 12) Synthesis of compound 14-15

Compound 14-14 (212 mg, 681.02 µmol) and hydrochloric acid/ethyl acetate (4 M, 1.34 mL) were added to a pre-dried single-necked flask, and the mixture was stirred at 30 °C for 2 hours. Saturated brine (2 mL) was added to the reaction solution, and the phases were separated, then the aqueous phase was collected; the pH of the aqueous phase was adjusted to 9 with saturated sodium hydroxide aqueous solution, and the mixture was extracted with ethyl acetate (2 mL^{∗}3); the phases were separated, and the organic phases were collected, dried over anhydrous sodium sulfate, filtered, and the filtrate was concentrated under reduced pressure to obtain crude compound 14-15.

### 13) Synthesis of compound 14A or 14B or 14C or 14D or 14E or 14F or 14G or 14H

Compound 14-15 (120 mg, 568.23 µmol), compound 1-14 (185 mg, 625.06 µmol), cesium carbonate (370 mg, 1.14 mmol), 2,2-bis(diphenylphosphino)-1,1-binaphthyl (35 mg, 56.82 µmol), tris(dibenzylideneacetone) dipalladium (52 mg, 56.82 µmol) were added to a pre-dried single-necked flask, then dioxane (2 mL) was added thereto, and the reaction system was evacuated and replaced with nitrogen for three times, then the mixture was stirred at 100 °C for 16 hours. After the mixture was concentrated under reduced pressure, ethyl acetate (20 mL) and saturated brine (10 mL) were added to separate the phases; the organic phase was dried over anhydrous sodium sulfate, filtered, and the filtrate was concentrated under reduced pressure to obtain a crude product; and the crude product was purified by column chromatography, purified by preparative high performance liquid chromatography (alkaline system) and resolved by supercritical fluid chromatography (alkaline system) in turn to obtain compound 14A. SFC detection (ee: 98.74 %), chromatographic column: Chiralpak AD-3 150 × 4.6 mm I.D., 3 µm; mobile phase: A: supercritical carbon dioxide, B: 0.05 % ethanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.90 min. ¹HNMR (400 MHz, CDCl₃) δ ppm 7.64 (d, *J*=8.7 Hz, 1H), 6.92 (d, *J*=2.3 Hz, 1H), 6.74 (dd, *J*=2.4, 8.7 Hz, 1H), 4.60 (dd, *J*=2.4, 10.7 Hz, 1H), 4.51 (dd, *J*=2.1, 8.3 Hz, 1H), 4.44-4.19 (m, 2H), 4.03-3.82 (m, 3H), 3.70 (dd, *J*=6.3, 10.8 Hz, 1H), 3.47 (dd, *J*=7.0, 10.4 Hz, 1H), 3.43-3.31 (m, 1H), 3.30-3.13 (m, 1H); LCMS (ESI) m/z: 381 [M+1]⁺.

### Embodiment 15

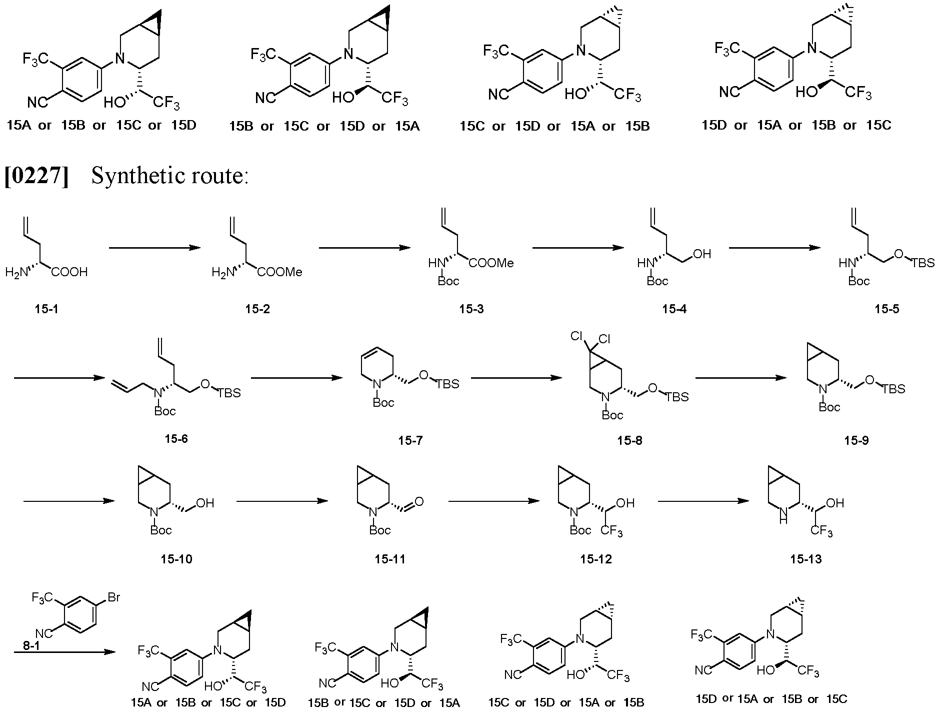

### 1) Synthesis of compound 15-2

Compound 15-1 (7.5 g, 65.14 mmol) was added to a three-necked flask, and hydrochloric acid/methanol (6 M, 100 mL) was added thereto, and the reaction system was replaced with nitrogen for three times, then the mixture was stirred at 70 °C for 4 hours. The reaction solution was concentrated under reduced pressure, then water (5 mL) was added thereto; the pH of the mixture was adjusted to 11 with saturated potassium carbonate solution, and the mixture was extracted with ethyl acetate (50 mL^{∗}3), then the organic phases were combined, washed with saturated brine (20 mL^{∗}3), dried over anhydrous sodium sulfate, filtered, and the filtrate was concentrated under reduced pressure to obtain crude compound 15-2. ¹HNMR (400 MHz, CDCl₃) δ ppm 5.69-5.83 (m, 1H), 5.12-5.20 (m, 2H), 3.74 (s, 3H), 3.58 (dd, *J*=7.45, 5.26 Hz, 1H), 2.48-2.57 (m, 1H), 2.35-2.47 (m, 1H).

### 2) Synthesis of compound 15-3

Compound 15-2 (7.3 g, 56.52 mmol), dichloromethane (80 mL), triethylamine (8.58 g, 84.78 mmol, 11.80 mL) were added to a three-necked flask, and the mixture was cooled to 0 °C; Boc anhydride (18.50 g, 84.78 mmol, 19.48 mL) was added thereto, and the mixture was naturally warmed to 25 °C and stirred for 1 hour. The reaction solution was washed with hydrochloric acid (1 M, 50 mL^{∗}3), and the organic phase was collected, dried over anhydrous sodium sulfate, filtered, and the filtrate was concentrated under reduced pressure to obtain a crude product, then the crude product was purified by column chromatography to obtain compound 15-3. ¹HNMR (400 MHz, CDCl₃) δ ppm 5.59-5.78 (m, 1H), 5.09-5.20 (m, 2H), 5.03 (br s, 1H), 4.39 (br d, *J*=7.03 Hz, 1H), 3.75 (s, 3H), 2.41-2.63 (m, 2H), 1.45 (s, 9H); LCMS (ESI) m/z: 130 [M-100+1]⁺.

### 3) Synthesis of compound 15-4

Compound 15-3 (5 g, 21.81 mmol), tetrahydrofuran (50 mL) were added to a there-necked flask, and the reaction system was replaced with nitrogen for three times, then the temperature was lowered to 0 °C; lithium borohydride (949.96 mg, 43.62 mmol) was added thereto, and the mixture was stirred at 0 °C for 2 hours. The reaction solution was quenched by adding saturated ammonium chloride solution (50 mL), extracted with ethyl acetate (50 mL^{∗}3); the organic phases were combined, washed with saturated brine (20 mL^{∗}3), dried over anhydrous sodium sulfate, filtered, and the filtrate was concentrated under reduced pressure to obtain a crude product, and the crude product was purified by column chromatography to obtain compound 15-4. ¹HNMR (400 MHz, CDCl₃) δ ppm 5.79 (ddt, *J*=17.10, 10.07, 7.18, 7.18 Hz, 1H), 5.06-5.21 (m, 2H), 4.50-4.81 (m, 1H), 3.54-3.80 (m, 3H), 2.17-2.38 (m, 2H), 1.45 (s, 9H); LCMS (ESI) m/z: 146 [M-55]⁺.

### 4) Synthesis of compound 15-5

Compound 15-4 (3.4 g, 16.89 mmol), *tert-*butyldimethylsilyl chloride (2.55 g, 16.89 mmol, 2.07 mL), imidazole (1.73 g, 25.34 mmol), dichloromethane (40 mL) were added to a round bottom flask, then the reaction system was replaced with nitrogen for three times, and the reaction was carried out at 25 °C for 2 hours. Ethyl acetate (30 mL) was added to the reaction solution, and the organic phase was washed with saturated brine (20 mL^{∗}3); the organic phase was collected, dried over anhydrous sodium sulfate, filtered, and the filtrate was concentrated under reduced pressure to obtain a crude product, and the crude product was purified by column chromatography to obtain compound 15-5. ¹HNMR (400 MHz, CDCl₃) δ ppm 5.71-5.89 (m, 1H), 5.01-5.17 (m, 2H), 3.55-3.71 (m, 3H), 2.16-2.43 (m, 2H), 1.45 (s, 9H), 0.91 (s, 9H), 0.06 (s, 6H).

### 5) Synthesis of compound 15-6

Compound 15-5 (4 g, 12.68 mmol), allyl bromide (2.30 g, 19.02 mmol), *N*,*N-*dimethylformamide (20 mL) were added to a three-necked flask, and the mixture was cooled to 0 °C, then a solution of potassium *tert*-butoxide (2.85 g, 25.35 mmol) in *N*,*N-*dimethylformamide (20 mL) was added thereto, and the mixture was stirred at 0 °C for 1 hour. Ethyl acetate (30 mL) was added to the reaction solution, and the organic phase was washed with saturated brine (20 mL^{∗}3); the organic phase was collected, dried over anhydrous sodium sulfate, filtered, and the filtrate was concentrated under reduced pressure to obtain a crude product, and the crude product was purified by column chromatography to obtain compound 15-6. ¹HNMR (400 MHz, CDCl₃) δ ppm 5.63-5.94 (m, 2H), 4.99-5.14 (m, 4H), 3.55-4.08 (m, 5H), 2.34 (br d, *J*=7.28 Hz, 2H), 1.38-1.53 (m, 9H), 0.90 (s, 9H), 0.01-0.11 (m, 6H); LCMS (ESI) m/z: 256 [M-100+1]⁺.

### 6) Synthesis of compound 15-7

Compound 15-6 (2.2 g, 6.19 mmol), dichloromethane (20 mL), Grubb catalyst I (benzylidene-bis(tricyclohexylphosphine)dichlororuthenium) (254.58 mg, 309.35 µmol) were added to a round bottom flask, and the reaction system was replaced with nitrogen for three times, then the reaction was carried out at 25 °C for 16 hours. The reaction solution was concentrated under reduced pressure to obtain a crude product, and the crude product was purified by column chromatography to obtain compound 15-7. ¹HNMR (400 MHz, CDCl₃) δ ppm 5.54-5.79 (m, 2H), 4.01-4.55 (m, 2H), 3.43-3.57 (m, 3H), 2.10-2.37 (m, 2H), 1.48 (s, 9H), 0.89 (s, 9H), 0.05 (d, *J*=1.76 Hz, 6H).

### 7) Synthesis of compound 15-8

Compound 15-7 (2.7 g, 8.24 mmol), benzyltriethylammonium chloride (375.53 mg, 1.65 mmol), chloroform (25 mL) were added to a three-necked flask, and 50 % sodium hydroxide aqueous solution (25 mL) was added dropwise thereto at 20 °C, then the system was kept at 15 °C and stirred for 1.5 hours. Water (40 mL) was added to the reaction solution, and the mixture was extracted with dichloromethane (50 mL^{∗}3); the organic phases were combined, washed with saturated brine (20 mL^{∗}3), dried over anhydrous sodium sulfate, filtered, and the filtrate was concentrated under reduced pressure to obtain a crude product, and the crude product was purified by column chromatography to obtain compound 15-8. ¹HNMR (400 MHz, CDCl₃) δ ppm 4.19 (br s, 1H), 3.97 (br d, *J*=13.68 Hz, 1H), 3.51-3.61 (m, 2H), 3.38 (dd, *J*=14.49, 6.71 Hz, 1H), 2.19 (br s, 1H), 1.63-1.93 (m, 3H), 1.44 (s, 9H), 0.90 (s, 9H), 0.07 (s, 6H); LCMS (ESI) m/z: 310 [M-100+1]⁺.

### 8) Synthesis of compound 15-9

Sodium (33.61 mg, 1.46 mmol) and tetrahydrofuran (5 mL) were added to a three-necked flask, and then a solution of compound 15-8 (0.2 g, 487.27 µmol) in methanol (5 mL) and tetrahydrofuran (5 mL) was added dropwise thereto, and the mixture was stirred at 25 °C for 2 hours. Water (5 mL) was added to the reaction solution, and the mixture was extracted with ethyl acetate (10 mL^{∗}3); the organic phases were combined, washed with saturated brine (5 mL^{∗}3), dried over anhydrous sodium sulfate, filtered, and the filtrate was concentrated under reduced pressure to obtain crude compound 15-9. ¹HNMR (400 MHz, CDCl₃) δ ppm 3.80 (br d, *J*=13.43 Hz, 1H), 3.49-3.69 (m, 2H), 3.31-3.43 (m, 1H), 1.82-2.31 (m, 1H), 1.51-1.82 (m, 2H), 1.39-1.51 (m, 9H), 0.90 (s, 9H), 0.62 (br s, 1H), 0.02-0.13 (m, 6H).

### 9) Synthesis of compound 15-10

Compound 15-9 (0.36 g, 1.05 mmol), tetrabutylammonium fluoride (1 M, 1.26 mL), and tetrahydrofuran (5 mL) were added to a round bottom flask, and the reaction system was replaced with nitrogen for three times, then the reaction was carried out at 25 °C for 2 hours. Ethyl acetate (10 mL) was added to the reaction solution, and the organic phase was washed with 1 M hydrochloric acid (10 mL^{∗}3), then the organic phase was collected, dried over anhydrous sodium sulfate, filtered, then the filtrate was concentrated under reduced pressure to obtain a crude product, and the crude product was purified by column chromatography to obtain compound 15-10. ¹HNMR (400 MHz, CDCl₃) δ ppm 4.01 (br s, 1H), 3.67-3.75 (m, 1H), 3.57-3.64 (m, 1H), 3.49 (br d, *J*=17.69 Hz, 1H), 1.88 (br d, *J*=14.31 Hz, 1H), 1.69-1.79 (m, 1H), 1.56 (br s, 1H), 1.47 (s, 9H), 0.84-1.05 (m, 2H), 0.56-0.71 (m, 1H), 0.15 (br s, 1H); LCMS (ESI) m/z: 172 [M-55]⁺.

### 10) Synthesis of compound 15-11

Compound 15-10 (70 mg, 307.96 mmol), ethyl acetate (2 mL), *N,N-*diisopropylethylamine (238.81 mg, 1.85 mmol, 321.84 µL) were added to a three-necked flask, and the reaction system was replaced with nitrogen for three times, then the mixture was cooled to 0 °C; a solution of pyridine sulfur trioxide (147.05 mg, 923.89 µmol) in dimethyl sulfoxide (2 mL) was added thereto, and the reaction was carried out at 0 °C for 1 hour. Ethyl acetate (2 mL) was added to the reaction solution, and the organic phase was washed with saturated brine solution (2 mL^{∗}3); the organic phase was collected, washed with 1 M hydrochloric acid (2 mL^{∗}3), and the organic phase was collected, dried over anhydrous sodium sulfate, filtered, and the filtrate was concentrated under reduced pressure to obtain crude compound 15-11. LCMS (ESI) m/z: 170 [M-55]⁺.

### 11) Synthesis of compound 15-12

Compound 15-11 (80 mg, 355.11 µmol), tetrahydrofuran (2 mL), and (trifluoromethyl)trimethylsilane (75.74 mg, 532.66 µmol) were added to a three-necked flask, and the mixture was cooled to 0 °C, then tetrabutylammonium fluoride (1 M, 710.22 µL) was added thereto, and the mixture was stirred at 0 °C for 2 hours. Ethyl acetate (3 mL) was added to the reaction solution, and the organic phase was washed with 1 M hydrochloric acid (3 mL^{∗}3), then the organic phase was collected, dried over anhydrous sodium sulfate, filtered, then the filtrate was concentrated under reduced pressure to obtain a crude product, and the crude product was purified by column chromatography to obtain compound 15-12. LCMS (ESI) m/z: 196 [M-100+1]⁺.

### 12) Synthesis of compound 15-13

Compound 15-12 (100 mg, 338.64 µmol), hydrochloric acid/ethyl acetate (6 M, 1 mL) were added to a thumb bottle, and the reaction was carried out at 0 °C for 1.5 hours. Water (5 mL) was added to the reaction system, and the phases were separated, then the aqueous phase was collected; the pH of the aqueous phase was adjusted to 11 with saturated potassium carbonate solution, and the mixture was extracted with ethyl acetate (10 mL^{∗}3), then the organic phases were combined, washed with saturated brine (5 mL^{∗}3), dried over anhydrous sodium sulfate, filtered, and the filtrate was concentrated under reduced pressure to obtain crude compound 15-13. ¹HNMR (400 MHz, MeOD) δ ppm 4.28-4.39 (m, 1H), 3.75 (ddd, J=13.48, 8.44, 4.82 Hz, 1H), 3.07-3.28 (m, 2H), 2.04-2.47 (m, 2H), 1.26-1.39 (m, 2H), 0.87-1.04 (m, 1H), 0.47-0.59 (m, 1H).

### 13) Synthesis of compound 15A

Compound 8-1 (51.24 mg, 204.94 µmol), compound 15-13 (40 mg, 204.94 µmol), cesium carbonate (166.93 mg, 512.34 µmol), tris(dibenzylideneacetone)dipalladium (18.77 mg, 20.49 µmol), 2,2-bis(diphenylphosphino)-1,1-binaphthyl (12.76 mg, 20.49 µmol), and dioxane (2 mL) were added to a thumb bottle, and the reaction system was replaced with nitrogen for three times, then the reaction was carried out at 100 °C for 2 hours. The reaction solution was concentrated under reduced pressure to obtain a crude product, then the crude product was purified by column chromatography, purified by preparative high performance liquid chromatography (acidic system) and resolved by supercritical fluid chromatography (alkaline system) in turn to obtain compound 15A. SFC detection (ee: 100 %), chromatographic column: Chiralpak AD-3 150 × 4.6 mm I.D., 3 µm; mobile phase: A: supercritical carbon dioxide, B: 0.05 % methanol solution of isopropylamine; gradient: held the initial 10 % of B for 0.5 min, from 10 % to 40 % in 2.0 min, held 40 % for 2.0 min, returned to 10 % in 0.7 min, equilibrated at 10 % for 0.8 min; flow rate: 2.5 mL/min; column temperature: 35 °C; wavelength: 220 nm, retention time: 1.45 min. ¹HNMR (400 MHz, CDCl₃) δ ppm 7.61 (d, J=8.66 Hz, 1H), 7.01 (d, *J*=2.38 Hz, 1H), 6.85 (dd, *J*=8.91, 2.64 Hz, 1H), 4.28-4.40 (m, 1H), 4.01-4.10 (m, 1H), 3.64-3.78 (m, 2H), 2.65 (br d, *J*=14.43 Hz, 1H), 2.41 (d, *J*=5.14 Hz, 1H), 1.82 (br d, *J*=14.18 Hz, 1H), 1.19 (br s, 2H), 0.81 (td, *J*=8.44, 5.21 Hz, 1H), 0.05-0.13 (m, 1H); LCMS (ESI) m/z: 364 [M+1]⁺.

### Experimental embodiment 1: agonistic effect of test compounds on androgen receptor (AR)

### 1. Preparation of compounds, placing in plates:

1.1 Preparation of compounds: the test compounds were diluted to the working concentration, and each compound was diluted by 4 times with Echo for 10 concentration gradients, and the compounds were added to a 384-well cell plate according to the microplate layout diagram, with 200 nL per well;
1.2 AR cell assay medium: 87 % Opti-MEM (reduced serum medium), 10 % Dialyzed FBS (dialyzed fetal bovine serum), 1 % NEAA (non-essential amino acids), 1 % sodium pyruvate and 1 % penicillin-streptomycin;
1.3 culture medium, trypsin and Dulbecco's phosphate buffer were preheated in a 37 °C water bath;
1.4 the original medium in the cell culture flask was removed, and the cells were washed once with 6 mL of Dulbecco's phosphate buffer;
1.5 3.5 mL of trypsin was added to the cell culture flask, and the flask was gently shaken to make the trypsin fully contact with the cells, the trypsin was removed; after aspirating the trypsin, the culture flask was placed in a 37 °C incubator containing 5 % CO₂ for about 1 minute;
1.6 the cells were resuspended with 10 mL of cell detection medium, and about 0.8 mL of cell suspension was taken out for counting (ViCell XR)

| Cell | Cell generation | Cell viability | Cell density (cells/mL) |
|---|---|---|---|
| AR | 20 | 95.1% | 1.53×10⁶ |

1.7 the cell suspension was diluted with culture medium to 2.5×10⁵ cells per mL;
1.8 40 µL of cell suspension was added to each well of the cell plate, and 40 µL of cell culture medium was added to the other wells, and the cells were incubated in a 37 °C incubator containing 5 % CO₂ for 16 hours.

### 2. Reading the board and analyzing data:

2.1 Preparation of solution A: 182 µL of DMSO was added to 200 µg of LiveBLAzer^{™}-FRET B/G/g substrate (CCF4-AM). The solution A was packaged and stored in a refrigerator at -20 °C;
2.2 preparation of 6×substrate buffer: 15 µL of solution A was added to 150 µL of solution B, and the mixture was vortexed evenly, then 2335 µL of solution C was added to the above solution and vortexed evenly;
2.3 the cell culture plate was taken out, and 8 µL of 6×substrate buffer was added to each well, shaken for 1 minute, and centrifuged at 1000 rpm for 10 seconds; after sealing the membrane, the cells were incubated at 23 °C for 2 hours, and the plate was read with Envision, and the curve EC₅₀ value was fitted by Prism software. The test results of the agonistic activity of the compound on androgen receptor (AR) are shown in Table 1 below.

**Table 1 Test results of agonist activity of compounds on androgen receptor**

| Number of the compound | EC₅₀ (nM ) | Number of the compound | EC₅₀ (nM) |
|---|---|---|---|
| Compound 1A | 0.81 | Compound 9A | 47.81 |
| Mixture of compounds 2A and 2B | 8.8 | Compound 10A | 55.78 |
| Compound 3A | 0.81 | Compound 11A | 135.3 |
| Compound 5A | 0.37 | Mixture of compounds 12A and 12B | 6.33 |
| Mixture of compounds 6A and 6B | 4.3 | Compound 14A | 0.39 |
| Compound 7A | 0.53 | Compound 15A | 0.22 |
| Compound 8A | 3.25 | | |

Experimental conclusion: the compounds of the present disclosure have significant agonistic activity on androgen receptor (AR).

### Experimental embodiment 2: pharmacokinetic test of the compounds of the present disclosure

### 1. Abstract

Male SD rats were used as test animals, and LC-MS/MS method was used to determine the drug concentrations in plasma of rats at different time after intravenous and intragastric administration of compound 1A, compound 3A and compound 8A. To study the pharmacokinetic behavior of the compounds in rats and to evaluate their pharmacokinetic characteristics.

### 2. Experimental scheme

2.1 Test drugs: compound 1A, compound 3A and compound 8A
2.2 Test animals: 12 healthy adult male SD rats, divided into 6 groups with 2 rats in each group. The animal was purchased from Shanghai Sippr-BK laboratory animal Co. Ltd., and the animal production license number was SCXK (Shanghai) 2013-0016.

### 2.3 Preparation of drugs

An appropriate amount of sample was weighed, and appropriate amounts of DMSO, polyethylene glycol-15 hydroxystearate and water were added in turn according to the volume ratio of 10:10:80, and the clarified state of 0.2 mg/mL was reached after stirring and sonication for intravenous administration.

An appropriate amount of sample was weighed and dissolved in 5 % Tween 80 + 90 % polyethylene glycol 400 + 5 % polyvinylpyrrolidone K30 solution, and the clarified state of 0.5 mg/mL was reached after stirring and sonication for intragastric administration.

### 2.4 Administration

12 male SD rats were divided into 6 groups, and after fasting overnight, groups 1-3 were administered intravenously with a volume of 5 mL/kg; groups 4-6 were administered by gavage with a volume of 10 mL/kg.

### 3. Operation

After compound 1A, compound 3A and compound 8A were intravenously administered to SD rats, 40 µL of blood was collected at 0.0833, 0.25, 0.5, 1, 2, 4, 8 and 24 hours, respectively, and placed in a test tube containing 2 µL of EDTA-K₂. After compound 1A and compound 3A were administered by gavage, 40 µL of blood was collected at 0.25, 0.5, 1, 2, 4, 8 and 24 hours, respectively, and placed in a test tube containing 2 µL of EDTA-K₂. The tubes were centrifuged at 4000 rpm for 15 minutes to separate the plasma and stored at - 60 °C. Animals were allowed to eat 2 hours after administration.

The content of the test compounds in the plasma of rats after intravenous and intragastric administration was determined by LC-MS/MS method. The linear range of the method was 2.00-6000 nmol/L; plasma samples were analyzed after acetonitrile precipitation protein treatment. The pharmacokinetic test results of compound 1A, compound 3A and compound 8A are shown in Table 2 below.

**Table 2 Pharmacokinetic test results of compound 1A, compound 3A and compound 8A**

| Test compound | Admini - stration method | Dosage of admini - stration mg/kg | Blood drug concen -tration | Peak time | Half -life | Apparent volume of distribution | Clearance rate | Curve area (0-t) | Curve area (0-inf) | Bioavailability |
|---|---|---|---|---|---|---|---|---|---|---|
| | | | Cmax (nM) | Tmax (h) | T_{1/2} (h) | Vdss (L/kg) | Cl (mL/min/ kg) | AUC₀₋ₗₐₛₜ (nM.h) | AUC_{0-inf} (nM.h) | F (%) |
| Compound 1A | Intravenous admini - stration | 1.1 | - | - | 1.37 | 1.43 | 20.6 | 2469 | 2502 | - |
| | Intragastric admini - strati on | 4.92 | 223 | 4.00 | ND | - | - | 1238 | ND | 11 |
| Compound 3A | Intravenous admini - stration | 0.69 | - | - | 1.35 | 1.83 | 28.6 | 1277 | 1289 | - |
| | Intragastric admini - strati on | 4.2 | 262 | 3.00 | 1.8 | - | - | 1247 | ND | 16 |
| | Intravenous | 0.716 | - | - | 4.18 | 2.22 | 6.99 | 4782 | 4871 | - |
| Compound 8A | admini - stration | | | | | | | | | |
| | Intragastric admini - strati on | 4.59 | 1660 | 4.00 | 3.69 | - | - | 12698 | 12895 | 41 |

| | | | | | | | | | | |
|---|---|---|---|---|---|---|---|---|---|---|
| Note: "-" indicates that the item does not need to be detected, and "ND" indicates that it is not detected. | | | | | | | | | | |

Experimental conclusion: the compounds of the present disclosure have a longer oral half-life, a certain oral exposure and oral bioavailability.

### Experimental embodiment 3: in vivo pharmacodynamic study of the compounds of the present disclosure for muscle growth in female rats

### 1. Experimental design

From 16 female SD rats, 12 were selected and randomly divided into 2 groups of 6 animals each according to their body weight, with 2 spare animals in each group; the vehicle was (5 % Tween 80 + 90 % polyethylene glycol 400 + 5 % polyvinylpyrrolidone K30): 1 % carboxymethyl cellulose = 1: 9 (v/v).

**Table 3 Experimental scheme of the compound of the present disclosure on the muscle growth model of female rats**

| Administration group | Compound | Volume of administration | Dosage of administration | Route of administration | Frequency of administration | Number of animals |
|---|---|---|---|---|---|---|
| 1 | Vehicle group | 5 mL/kg | - | Oral | QD × 37 days | 6 |
| 2 | Compound 3A | 5 mL/kg | 10 mg/kg | Oral | QD × 37 days | 6 |

### 2. Experimental Materials:

2.1 Experimental animals
Species: rat
Strain: SD rats, SPF grade
Age and weight: 11-16 weeks old, weight 250-350 g
Gender: female
Supplier: Beijing Vital River Laboratory Animal Technology Co., Ltd.
Animal license number: GP02-091-2019v1.0

### 3. Experimental methods and procedures

The rats were acclimatized in the ecological feeding box for 1 week at approximately 2 months of age, and after the acclimatization period, the rats were divided equally into 2 groups (n=6) according to body weight, and the vehicle and compound 3A were administered by gavage, once a day, for 5 weeks, at which time the animals were euthanized, and the tissues (muscle tissue: levator anal muscle, vaginal smooth muscle and gastrocnemius; gonad-related tissue: clitoris) were collected by necropsy and weighed.

### 4. Experimental results

Compared with the vehicle group, compound 3A increased animal body weight by 20 % and animal muscle weight by 34 %; while compound 3A increased clitoral weight by 19 %, which was significantly lower than the effect on muscle weight gain, indicating that compound 3A had little effect on gonad organs and low potential side effects while maintaining the weight gain effect of muscle. The animals in all groups showed no significant abnormalities and were well tolerated.

Experimental conclusion: compound 3A can significantly increase muscle weight and animal body weight in female rat muscle growth pharmacodynamic model, and has little effect on gonadal organs, and its potential side effects are low.

### Experiment embodiment 4: human liver microsomal CYP inhibition experiment

### Experimental purposes:

The inhibitory effects of the test compound 3A and VK5211 on the activities of human liver microsomal cytochrome P450 isozymes (CYP1A2, CYP2C9, CYP2C19, CYP2D6 and CYP3A4) were determined.

Mixed human liver microsomes (HLM) were purchased from Corning Inc. (Steuben, New York, USA) or XenoTech, LLC. (Lenexa, KS, USA) or other suppliers, and were stored at a temperature below -60 °C before use.

### Experimental operation:

First, the test compound (10.0 mM) was gradient diluted to prepare working solution (100× final concentration), and the concentrations of the working solution were: 5.00, 1.50, 0.500, 0.150, 0.0500, 0.0150, 0.00500 mM, and a working solution of each positive inhibitor of P450 isozymes (CYP1A2, CYP2C9, CYP2C19, CYP2D6, and CYP3A4) and a specific substrate mixture thereof (5 in 1) were prepared at the same time; human liver microsomes stored in a refrigerator at a temperature lower than -60 °C were put on ice to thaw, and when all the human liver microsomes were dissolved, the microsomes were diluted with potassium phosphate buffer (PB) to prepare a working solution of a certain concentration (0.253 mg/mL). 20.0 µL of probe substrate mixed solution was firstly added to a reaction plate (20.0 µL of PB was added to a Blank well), then 158 µL of human liver microsomal working solution was added to the reaction plate, and the reaction plate was placed on ice for use; at this time, 2.00 µL of each concentration of the test compound (N=1) and a specific inhibitor (N=2) were added to a corresponding well, and a corresponding organic solvent was added to a group without inhibitor (test compound or positive inhibitor) as a control group sample (the test compound control sample was 1:1 DMSO: MeOH, and the positive control sample was 1: 9 DMSO: MeOH); after preincubation in a water bath at 37 °C for 10 min, 20.0 µL of a coenzyme factor (NADPH) solution was added to a reaction plate, and placed in a water bath at 37 °C for 10 min for incubation; 400 µL of a pre-cooled acetonitrile solution (containing an internal standard of 200 ng/mL Tolbutamide and Labetalol) was added to stop the reaction; the reaction plate was placed on a shaker and shaken and mixed evenly for 10 min; then the mixture was centrifuged at 4 °C and 4000 rpm for 20 min; 200 µL of supernatant was taken and added to 100 µL of water for sample dilution; finally, the plate was sealed, shaken, and mixed evenly for LC/MS/MS detection. The concentration of metabolites produced by the probe substrate in the sample was determined by liquid chromatography-tandem mass spectrometry (LC-MS/MS) method. Non-linear regression analysis of the percentage activity of the test sample on the concentration was performed using SigmaPlot (V.14) or XLfit software. The IC₅₀ values were calculated by a three- or four-parameter inverse logarithmic equation. The experimental results are shown in Table 4:

**Table 4. Results of the inhibitory effect of test compounds on human liver microsomal cytochrome P450 isoenzyme activity**

| Test compound | IC₅₀ (µM) | | | | |
|---|---|---|---|---|---|
| | CYP1A2 | CYP2C9 | CYP2C19 | CYP2D6 | CYP3A4 |
| 3A | 29.3 | >50 | 12.1 | >50 | >50 |
| VK5211 | 4.44 | 5.84 | 4.23 | 7.67 | 44.7 |
| Comparative embodiment 1 | 11.5 | 6.4 | 0.336 | 14.3 | >50 |

Experimental conclusion: the compounds of the present disclosure have low risk of drug combination.

## Claims

1. A compound represented by formula (I) or a pharmaceutically acceptable salt thereof, 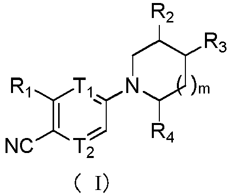 wherein,
T₁ is independently selected from N, CH and CR₅;
T₂ is independently selected from N, CH and CR₆;
R₁ is independently selected from H, F, Cl, Br, I, OH, NH₂, CN, C₁₋₃ alkyl and C₁₋₃ alkoxy, and the C₁₋₃ alkyl and C₁₋₃ alkoxy are optionally substituted by 1, 2 or 3 Rₐ;
R₂ and R₃ are each independently selected from F, Cl, Br, I, OH and NH₂;
or, R₂ and R₃ combining with the atoms to which they are attached form C₃₋₅ cycloalkyl and tetrahydrofuranyl, and the C₃₋₅ cycloalkyl and tetrahydrofuranyl are optionally substituted by 1, 2 or 3 R_{b};
m is 0, 1 or 2;
R₄ is independently selected from F, Cl, Br, I, OH, C₁₋₆ alkyl and C₁₋₆ alkoxy, and the C₁₋₆ alkyl and C₁₋₆ alkoxy are optionally substituted by 1, 2 or 3 R_{c};
R₅ is independently selected from F, Cl, Br, I, CN, C₁₋₃ alkyl and C₁₋₃ alkoxy, and the C₁₋₃ alkyl and C₁₋₃ alkoxy are optionally substituted by 1, 2 or 3 R_{d};
R₆ is independently selected from F, Cl, Br, I, OH, NH₂ and CN;
Rₐ, R_{b} and R_{d} are each independently selected from F, Cl, Br, I and OH;
R_{c} is independently selected from F, Cl, Br, I, OH, C₁₋₃ alkyl and C₁₋₃ alkoxy, and the C₁₋₃ alkyl and C₁₋₃ alkoxy are optionally substituted by 1, 2 or 3 R;
R is independently selected from F, Cl, Br and I.

2. The compound as claimed in claim 1 or the pharmaceutically acceptable salt thereof, wherein, R₁ is independently selected from H, F, Cl, Br, I, OH, NH₂, CN, CH₃, CH₂CH₃, C(CH₃)₂ and OCH₃, and the CH₃, CH₂CH₃, C(CH₃)₂ and OCH₃ are optionally substituted by 1, 2 or 3 Rₐ.

3. The compound as claimed in claim 2 or the pharmaceutically acceptable salt thereof, wherein, R₁ is independently selected from H, F, Cl, Br, I, OH, NH₂, CN, CH₃, CH₂F, CHF₂, CF₃, CH₂CH₃, C(CH₃)₂, OCH₃ and OCHF₂.

4. The compound as claimed in any one of claims 1 to 3 or the pharmaceutically acceptable salt thereof, wherein, R₂ and R₃ combining with the atoms to which they are attached form cyclopropyl, cyclobutyl, cyclopentyl and tetrahydrofuranyl, and the cyclopropyl, cyclobutyl, cyclopentyl and tetrahydrofuranyl are optionally substituted by 1, 2 or 3 R_{b}.

5. The compound as claimed in claim 4 or the pharmaceutically acceptable salt thereof, wherein, R₂ and R₃ combining with the atoms to which they are attached form

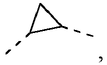

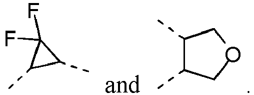

6. The compound as claimed in any one of claims 1 to 3 or the pharmaceutically acceptable salt thereof, wherein, R_{c} is independently selected from F, Cl, Br, I, OH, CH₃, CH₂F, CHF₂, CF₃, CH₂CH₃, C(CH₃)₂ and OCH₃.

7. The compound as claimed in claim 6 or the pharmaceutically acceptable salt thereof, wherein, R₄ is independently selected from F, Cl, Br, I, OH, NH₂, CN, C₁₋₃ alkyl and C₁₋₃ alkoxy, and the C₁₋₃ alkyl and C₁₋₃ alkoxy are optionally substituted by 1, 2 or 3 R_{c}.

8. The compound as claimed in claim 7 or the pharmaceutically acceptable salt thereof, wherein, R₄ is independently selected from F, Cl, Br, I, OH, CH₃, CH₂CH₃ and OCH₃, and the CH₃, CH₂CH₃ and OCH₃ are optionally substituted by 1, 2 or 3 R_{c}.

9. The compound as claimed in claim 8 or the pharmaceutically acceptable salt thereof, wherein, R₄ is independently selected from F, Cl, Br, I, OH, CH₃, CF₃, CH₂CH₃, OCH₃ and

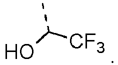

10. The compound as claimed in claim 9 or the pharmaceutically acceptable salt thereof, wherein, R₄ is independently selected from

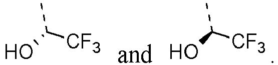

11. The compound as claimed in any one of claims 1 to 3 or the pharmaceutically acceptable salt thereof, wherein, R₅ is independently selected from F, Cl, Br, I, CN, CH₃ and OCH₃, and the CH₃ and OCH₃ are optionally substituted by 1, 2 or 3 R_{d}.

12. The compound as claimed in claim 11 or the pharmaceutically acceptable salt thereof, wherein, R₅ is independently selected from F, Cl, Br, I, CN, CH₃ and OCH₃.

13. The compound as claimed in claim 1 or the pharmaceutically acceptable salt thereof, wherein, the structure moiety 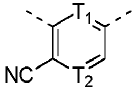 is selected from 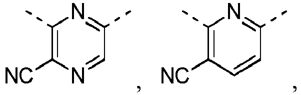 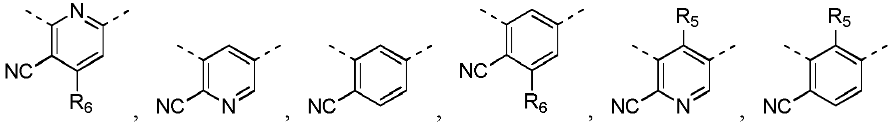 and

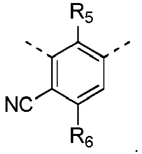

14. The compound as claimed in claim 13 or the pharmaceutically acceptable salt thereof, wherein, the structure moiety 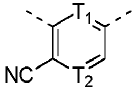 is selected from

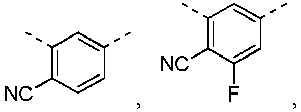

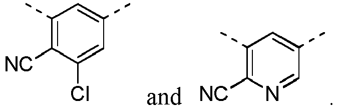

15. The compound as claimed in any one of claims 1-12 or the pharmaceutically acceptable salt thereof, selected from: 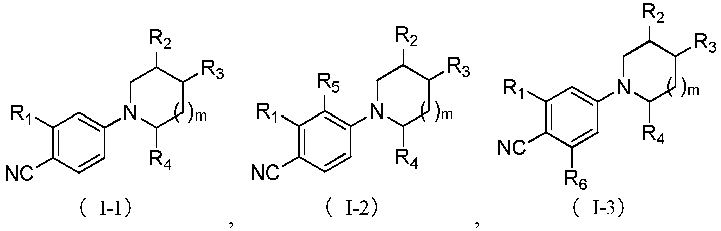 and 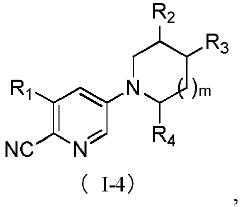 wherein,
R₁ is as defined in claims 1-3;
R₂, R₃ are as defined in claim 1, 4 or 5;
R₄ is as defined in claim 1, 7-10;
R₅ is as defined in claim 1, 11 or 12;
R₆ and m are as defined in claim 1.

16. The compound as claimed in claim 15 or the pharmaceutically acceptable salt thereof, which is selected from: 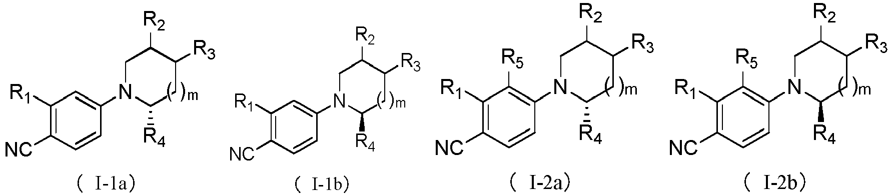 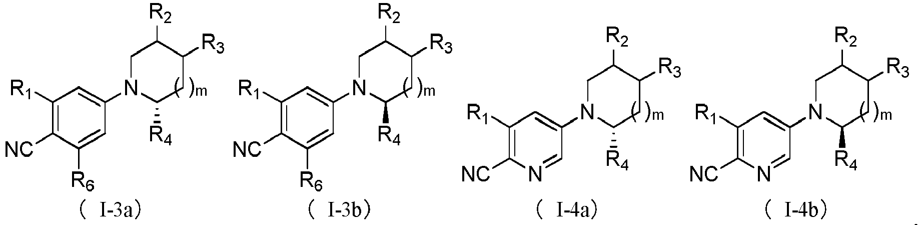 wherein,
R₁, R₂, R₃, R₄, R₅, R₆ and m are as defined in claim 15.

17. A compound represented by the following formula or a pharmaceutically acceptable salt thereof,

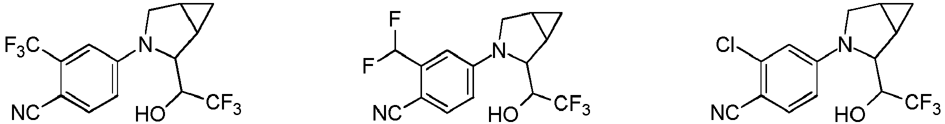

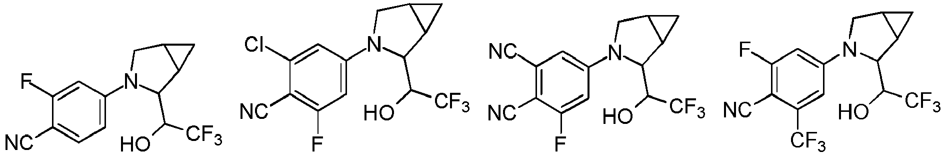

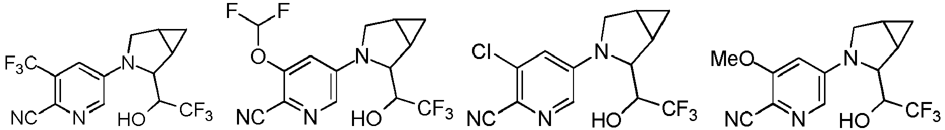

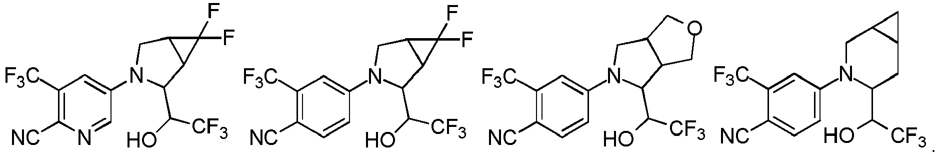

18. The compound as claimed in claim 17 or the pharmaceutically acceptable salt thereof, which is selected from:

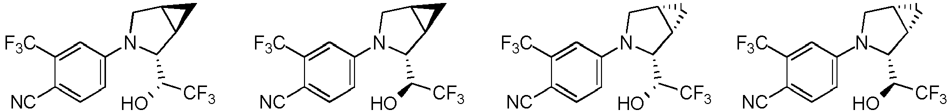

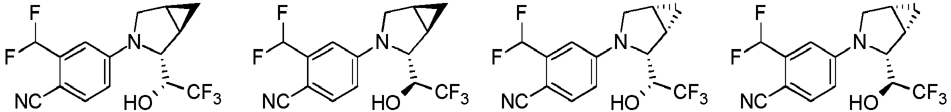

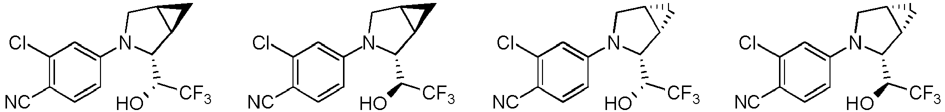

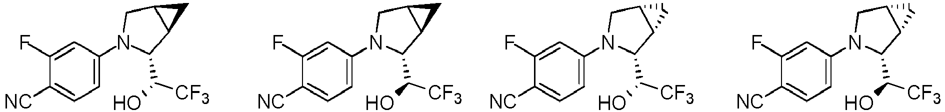

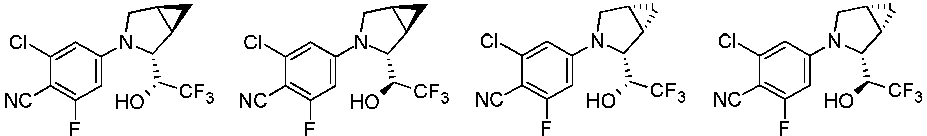

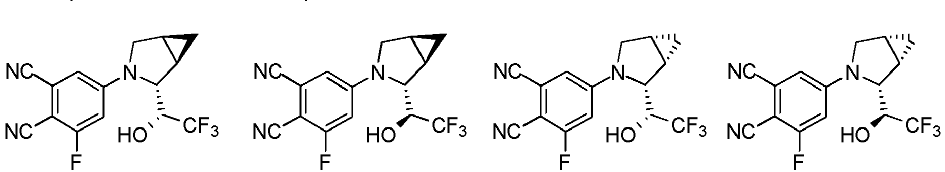

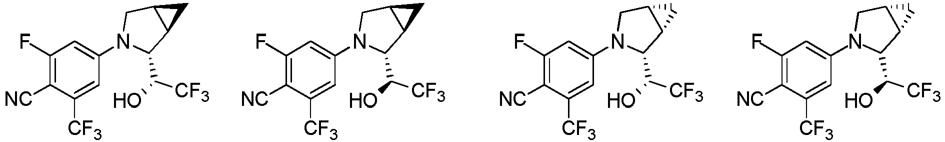

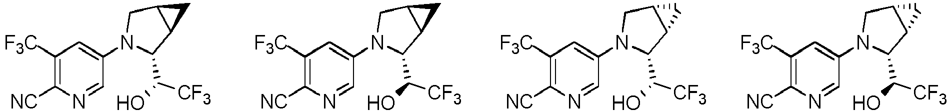

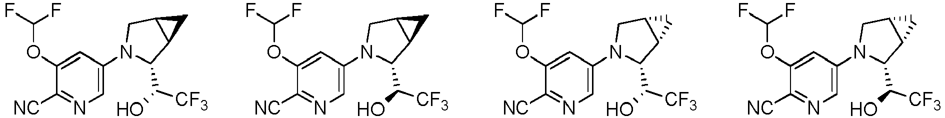

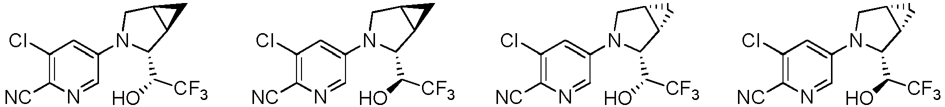

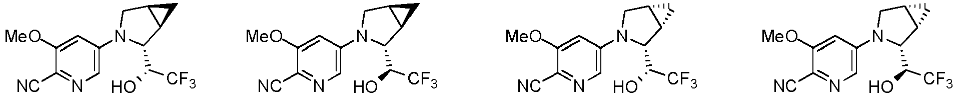

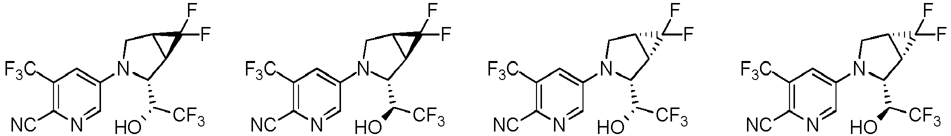

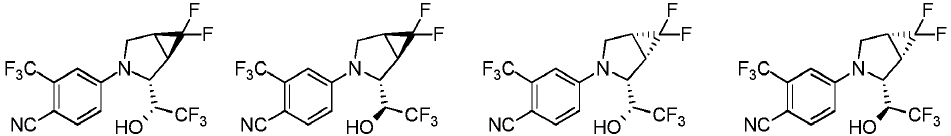

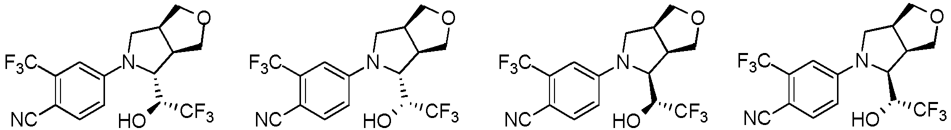

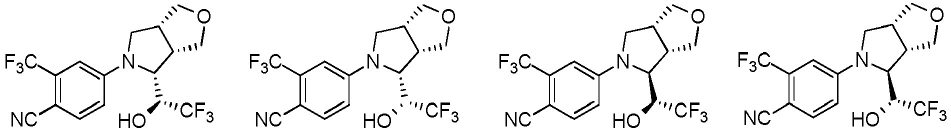

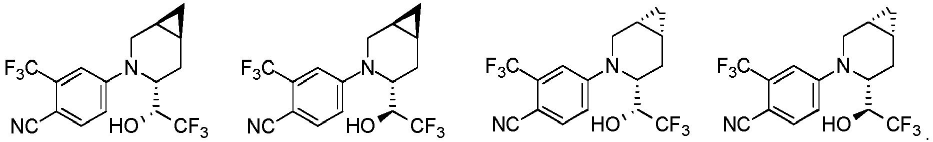

19. Use of the compound as claimed in any one of claims 1-18 or the pharmaceutically acceptable salt thereof in the manufacture of a medicament for the treatment of related diseases mediated by androgen receptor.

20. The use as claimed in claim 19, wherein, the medicament is a medicament for various senile diseases such as muscle atrophy, fractures, osteoporosis and the like.
